# EUROPEAN PATENT APPLICATION

(11) **EP 3 623 809 A1**
(43) Date of publication of application: **18.03.2020**
(21) Application number: 18798935.5
(22) Date of filing: 11.05.2018
(51) Int. Cl.: G01N 33/50, C07K 1/22, C07K 14/42, C07K 14/46, C12M 1/00, C12M 1/26, C12M 1/34, C12Q 1/68, G01N 33/574

(54) **CANCER DIAGNOSIS DEVICE**

(30) Priority: 12.05.2017 JP 2017096018
(71) Applicant: Hiroshima University, Higashi-Hiroshima-shi Hiroshima 739-8511 (JP); Kagoshima University, Kagoshima-shi, Kagoshima 890-8580 (JP); Alps Alpine Co., Ltd., Tokyo 1458501 (JP)
(72) Inventor: HORI, Kanji, Higashi-Hiroshima-shi Hiroshima 739-8528 (JP); HIRAYAMA, Makoto, Higashi-Hiroshima-shi Hiroshima 739-8528 (JP); MARUYAMA, Ikuro, Kagoshima-shi Kagoshima 890-8580 (JP); TAGUCHI, Yoshihiro, Tokyo 1458501 (JP); KUROKAWA, Hiroshi, Tokyo 145-8501 (JP); KOBAYASHI, Kenya, Tokyo 145-8501 (JP); MINAKUCHI, Hiroyoshi, Kyoto-shi Kyoto 602-8155 (JP)
(74) Representative: Hoffmann Eitle
(86) International application number: PCT/JP2018/018415
(87) International publication number: WO 2018/207932

(57) **Abstract**

To provide a device capable of cancer diagnosis with high sensitivity and specificity, a cancer diagnosis device (1) includes: an extracellular vesicle capturing section (16 to 18) including immobilization supports on which lectins are immobilized respectively, the lectins being each capable of binding specifically to a surface sugar chain included in an extracellular vesicle derived from a cancer cell, the immobilization supports corresponding respectively to one or more kinds of the surface sugar chain; and a detecting section configured to detect a microRNA included in the extracellular vesicle.

## Description

### Technical Field

The present invention relates to a cancer diagnosis device.

### Background Art

Cancer has been Japan's first leading cause of death since 1981. It is important to, for example, reduce the risk of cancer and increase the healthy life expectancy. Prognosis becomes poorer as the stage of cancer becomes later. If cancer has been discovered at stage 1, the subject can almost fully recover. It is important to have a technique of discovering cancer at a stage as early as possible. There is a particular demand for diagnosing cancer early with use of a sample with which diagnosis can be carried out easily such as blood and urine.

From the above viewpoint, there have been efforts made to search for various cancer markers to detect cancer at an early stage. Such markers, however, have very low sensitivity and cannot be used to diagnose early cancer. The markers are used merely to monitor detected cancer. The markers also do not have sufficient specificity. The concentration of such a marker increases even with a disease other than cancer. In addition, the concentration of a single cancer marker can increase with different types of cancer, which makes it impossible to determine the type of cancer.

Recent years have seen efforts to analyze a microRNA contained in an extracellular vesicle for early cancer diagnosis and/or monitoring for recurrence. An extracellular vesicle secreted from a cancer cell is present stably in a body fluid such as blood and urine. Examining a plurality of microRNAs contained in an extracellular vesicle makes it possible to (i) discover initial occurrence, recurrence, and metastasis of cancer at a stage where the cancer is still small and (ii) determine in which organ the cancer is present.

Non-Patent Literature 1, for example, studies the characteristics of a circulating microRNA that can be detected in major types of cancer such as lung cancer and gastric cancer. The results of the study provide the expectation that a circulating microRNA can serve as a marker for a cancer cell.

An extracellular vesicle derived from cancer is known as being related to formation of metastasis destination niche, which promotes the growth of cancer cells at the metastatic destination of the cancer. It is expected that analyzing a microRNA of an extracellular vesicle in blood will make it possible to predict metastasis destination of cancer.

A known conventional technique for separating and quantifying an exosome (which is a type of extracellular vesicle) is a method including (i) a step of bringing a sample containing an exosome into contact with a lectin (such as GNA) fixed to a base, (ii) a step of bringing into contact with the exosome a detectable pharmaceutical drug that binds to the exosome, and (iii) detecting a signal from the pharmaceutical drug to quantify the exosome (Patent Literature 1).

To efficiently separate an exosome and/or a target protein expressed in a cancer cell or the like, the inventors of the present invention have developed a protein separating device that specifically recognizes a protein contained in an analyte which protein has a high-mannose sugar chain (Patent Literature 2).

### Citation List

### [Patent Literature]

[Patent Literature 1]
   Specification of US Patent Application Publication No. 2013/0323756 (Publication Date: December 5, 2013)
[Patent Literature 2]
   Japanese Patent Application Publication, *Tokukai,* No. 2016-147839 (Publication Date: August 18, 2016)

### [Non-patent Literature]

[Non-patent Literature 1]
Kai Wang et al., Clinical Chemistry, pp. 1138-1155, 2015

### Summary of Invention

### Technical Problem

Extracellular vesicles are secreted by cells other than cancer cells such as immune cells and other normal cells. Further, early cancer is small. Juvenile cancer cells secrete extracellular vesicles into blood or the like in only a small amount, and such extracellular vesicles have a low concentration in blood. Extracellular vesicles in blood have a very small proportion of extracellular vesicles derived from cancer cells. This has made it still difficult to diagnose cancer early as desired.

In addition, microRNAs, which can increase or decrease in blood significantly, often correspond to two or more types of cancer. Determining the type of cancer requires detecting a microRNA in a trace amount that increases or decreases by only a small amount. It has been still difficult for the above reasons to discover initial occurrence, recurrence, and metastasis of cancer organ-specifically at a stage where the cancer is small.

Extracellular vesicles can be concentrated by ultracentrifugation or with use of an antibody. Such concentration, however, does not distinguish between extracellular vesicles derived from cancer cells and those secreted by normal cells. MicroRNAs in a trace amount derived from cancer cells are buried among microRNAs derived from other cells. This has made it impossible to improve the sensitivity and specificity as expected.

Development has been underway of a technique of (i) extracting from a body fluid an extracellular vesicle derived from a cancer cell and (ii) analyzing a microRNA contained in the extracellular vesicle as above to make it possible to diagnose, for example, initial occurrence, recurrence, and metastasis of cancer early. There has thus been a demand for more specificity in separating and concentrating the extracellular vesicle. No technique has unfortunately been available for meeting the demand.

The present invention has been accomplished in view of the above issue. It is an object of the present invention to provide a device capable of (i) specifically separating and concentrating an extracellular vesicle contained in a body fluid or the like and derived from a cancer cell and (ii) detecting a microRNA contained in the separated and concentrated extracellular vesicle to diagnose cancer with high sensitivity and specificity.

### Solution to Problem

In order to attain the above object, the inventors of the present invention conducted diligent research, and have thereby discovered that using a lectin that can bind specifically to a surface sugar chain of a protein included in an extracellular vesicle derived from a cancer cell allows, not an extracellular vesicle derived from a normal cell, but the above extracellular vesicle to be captured and concentrated selectively for high-sensitivity cancer diagnosis.

The inventors of the present invention have also discovered that specificity in diagnosis can be improved through analysis of the pattern of the sugar chain structure of the surface sugar chain and the pattern of how microRNAs contained in an extracellular vesicle are present. The inventors of the present invention have thereby completed the present invention.

Further, separately extracting a microRNA present in a microparticle and a microRNA present in an exosome in extracting microRNAs from extracellular vesicles captured allows information to be obtained on the microRNA present in each extracellular vesicle. The inventors of the present invention have discovered that combining the above information with information on the sugar chain structure of the surface sugar chain allows for further improvement in the specificity in diagnosis. The inventors of the present invention have thereby completed the present invention. Specifically, the present invention covers the inventions below.
[1] A cancer diagnosis device, including: an extracellular vesicle capturing section including one or more immobilization supports on which one or more kinds of lectins are immobilized respectively, the one or more kinds of lectins being each capable of binding specifically to a surface sugar chain included in an extracellular vesicle derived from a cancer cell, the one or more immobilization supports corresponding respectively to one or more kinds of the surface sugar chain, the extracellular vesicle capturing section being configured to capture the extracellular vesicle through specific binding to a corresponding one of the one or more kinds of lectins; and a detecting section configured to detect a microRNA included in the extracellular vesicle.
[2] The cancer diagnosis device according to [1], wherein the extracellular vesicle is an exosome and/or a microparticle.
[3] The cancer diagnosis device according to [1] or [2], wherein the one or more immobilization supports are each a monolithic gel or a lectin-solid-phased magnetic bead.
[4] The cancer diagnosis device according to [3], wherein the monolithic gel is a silica monolith.
[5] The cancer diagnosis device according to any one of [1] to [4], further including: an introduction section configured to introduce a sample into the extracellular vesicle capturing section; one or more discharge sections each configured to discharge liquid eluted from the extracellular vesicle capturing section and including the microRNA, the extracellular vesicle capturing section being positioned between the introduction section and the one or more discharge sections; a flow path between the introduction section and the extracellular vesicle capturing section; and a flow path between the extracellular vesicle capturing section and each of the one or more discharge sections, wherein the one or more discharge sections correspond in number to the one or more immobilization supports.
[6] The cancer diagnosis device according to any one of [1] to [5], wherein: the one or more kinds of lectins are one or more kinds of high-mannose sugar chain binding lectins in a case where the surface sugar chain is a high-mannose sugar chain, one or more kinds of sialyl Lewis sugar chain binding lectins in a case where the surface sugar chain is a sialyl Lewis sugar chain, or one or more kinds of core α1-6 fucose binding lectins in a case where the surface sugar chain is a core α1-6 fucose.
[7] The cancer diagnosis device according to [6], wherein the one or more kinds of high-mannose sugar chain binding lectins are one or more kinds of lectins selected from the group consisting of Solnin A, Solnin B, Solnin C, ESA-1, ESA-2, EAA-1, EAA-2, EAA-3, EDA-1, EDA-2, EDA-3, ECA-1 (KAA-1), ECA-2 (KAA-2), KAA-3, KSA-1, KSA-2, MPA-1, MPA-2, Granin-BP, ASL-1, ASL-2, OAA, BCA, BPL17, BML17, BCL17, and MPL-1.
[8] The cancer diagnosis device according to [6], wherein the one or more sialyl Lewis sugar chain binding lectins are each a selectin.
[9] The cancer diagnosis device according to [6], wherein the one or more kinds of core α1-6 fucose binding lectins are one or more kinds of lectins selected from the group consisting of Hypnin A-1, Hypnin A-2, and Hypnin A-3.
[10] The cancer diagnosis device according to any one of [1] to [9], wherein: the extracellular vesicle is included in one or more kinds of samples selected from the group consisting of blood, blood plasma, blood serum, saliva, tear, swab, phlegm, urine, spinal fluid, amniotic fluid, synovial fluid, ascitic fluid, and pleural fluid.

### Advantageous Effects of Invention

An aspect of the present invention advantageously provides a device capable of cancer diagnosis with highly sensitive and specificity.

### Brief Description of Drawings

Fig. 1 is a diagram schematically illustrating respective example configurations of the extracellular vesicle capturing section and the microRNA accommodating section, which are included in a cancer diagnosis device in accordance with an embodiment of the present invention.
Fig. 2 is a diagram schematically illustrating an example of how extracted liquid extracted from extracellular vesicle capturing sections is fed to an array to which cDNA of microRNAs is fixed as probes.
Fig. 3 is a diagram schematically illustrating an example of how cancer diagnosis is carried out on the basis of the result of observation of fluorescence from the array illustrated in Fig. 2.
Fig. 4 indicates results of SDS-PAGE carried out in Example 1.
Fig. 5 indicates results of a Western blot, using anti-CD9 antibody as a primary antibody, in Example 1.
Fig. 6 indicates results of a Western blot, using anti-CD63 antibody as a primary antibody, in Example 1.
Fig. 7 indicates results of comparing amounts of microRNA contained in fractions in which exosomes are collected, in Example 2.
Fig. 8 shows an example structure of a high-mannose sugar chain.
Fig. 9 illustrates a procedure used for capturing extracellular vesicles in Example 4.
Fig. 10 indicates results of Western blots carried out with use of anti-CD9 antibody and anti-CD81 antibody, for ultracentrifugation fractions derived from six types of cancer cells.
Fig. 11 indicates results of a Western blot, using anti-CD9 antibody, and immunostaining, carried out for e.g. eluates of AIST-OAA1 columns to which extracellular vesicle fractions derived from cancer cells has been added.
Fig. 12 indicates results of a relative quantitative analysis of captured and collected extracellular vesicles.
Fig. 13 illustrates properties of extracellular vesicles of A375. (a) of Fig. 13 illustrates a procedure for preparing an ultracentrifugation fraction. (b) of Fig. 13 indicates results of a Western blot carried out for the ultracentrifugation fraction. (c) of Fig. 13 indicates results of a nanoparticle tracking analysis of the ultracentrifugation fraction (d) of Fig. 13 indicates results of TEM observation of the ultracentrifugation fraction.
Fig. 14 indicates results of fractionating, by HPLC, sugar chains contained in extracellular vesicles. (a) of Fig. 14 indicates results of fractionating, by HPLC, the sugar chains which have been labeled with PA. (b) of Fig. 14 indicates the results of evaluating, by dual gradient reverse phase HPLC, the quantity of the sugar chains labeled with PA.
(a) of Fig. 15 illustrates a structure of a high-mannose N-type sugar chain. (b) of Fig. 15 indicates results of a Western blot carried out for an ultracentrifugation fraction treated with endoglycosidase H.
(a) of Fig. 16 indicates results of Western blots carried out for, e.g., an ultracentrifugation fraction treated with endoglycosidase H. (b) of Fig. 16 indicates results of quantifying the results shown in (a) of Fig. 16.

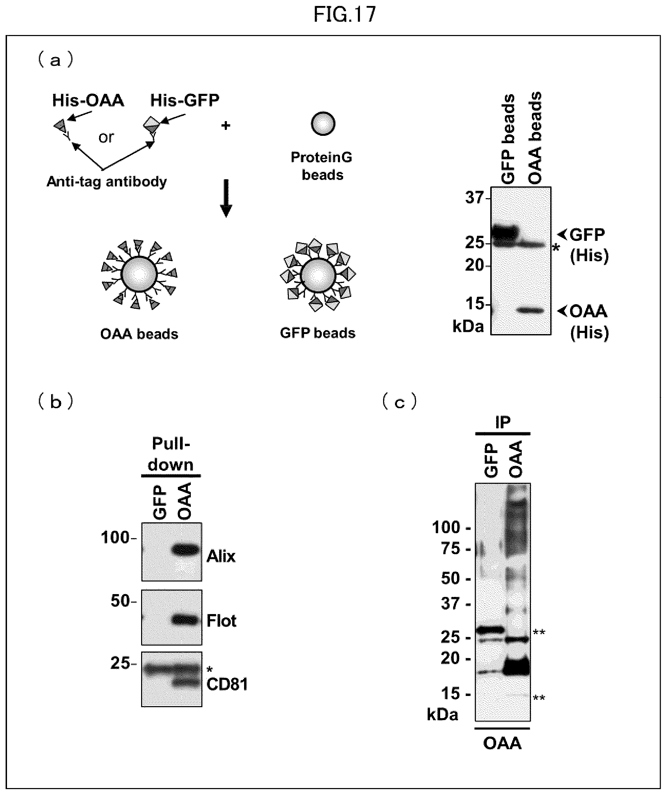

The left side of (a) of Fig. 17 is a diagram schematically illustrating a method of preparing OAA solid-phased magnetic beads. The right side of (a) of Fig. 17 indicates results of a Western blot carried out for an antibody-OAA complex and an antibody-GFP complex. (b) of Fig. 17 indicates results of a pulldown assay of extracellular vesicles derived from A375, carried out with use of OAA solid-phased magnetic beads and GFP solid-phased magnetic beads. (c) of Fig. 17 indicates results of immunoprecipitation of components captured by the OAA solid-phased magnetic beads and the GFP solid-phased magnetic beads.

### Description of Embodiments

The following description will discuss details of the present invention. The scope of the present invention is, however, not limited to this description. Besides the examples below, the present invention can also be modified and put into practice as appropriate within the range of not impairing the purpose of the present invention.

In the present specification, any range "A to B" means "not less than A and not more than B". Further, the patent literatures and non-patent literatures cited in the present specification are incorporated herein by reference.

### [1. Cancer diagnosis device]

A cancer diagnosis device in accordance with an embodiment of the present invention includes an extracellular vesicle capturing section including one or more immobilization supports on which one or more kinds of lectins are immobilized respectively, the one or more kinds of lectins being each capable of binding specifically to a surface sugar chain included in an extracellular vesicle derived from a cancer cell, the one or more immobilization supports corresponding respectively to one or more kinds of the surface sugar chain, the extracellular vesicle capturing section being configured to capture the extracellular vesicle through specific binding to a corresponding one of the one or more kinds of lectins; and a detecting section configured to detect a microRNA included in the extracellular vesicle.

### <1-1. Extracellular vesicle capturing section>

### (A) Extracellular vesicle, surface sugar chain, lectin

As described earlier, cancer cells and normal cells secrete extracellular vesicles into, for example, blood. Examples of the extracellular vesicles include an exosome, a microparticle, and an apoptotic body. An extracellular vesicle derived from a cancer cell includes a protein having a surface sugar chain specific to that extracellular vesicle. The "surface sugar chain" is a sugar chain bound to the surface of the protein molecules.

Examples of the surface sugar chain include (i) a high-mannose sugar chain included in an extracellular vesicle derived from a breast cancer cell, a prostate cancer cell, or a melanoma cell, (ii) a core α1-6 fucose included in an extracellular vesicle derived from a liver cancer cell, and (iii) a sialyl Lewis sugar chain derived from a pancreas cancer cell, a gastric cancer cell, or a lung cancer cell.

The extracellular vesicle capturing section includes one or more immobilization supports on which one or more kinds of lectins are immobilized respectively, the one or more kinds of lectins being each capable of binding specifically to a surface sugar chain included in an extracellular vesicle derived from a cancer cell, the one or more immobilization supports corresponding respectively to one or more kinds of the surface sugar chain. With this arrangement, the specific binding between the surface sugar chain and the lectin allows an extracellular vesicle derived from a cancer cell to be selectively collected from a sample such as blood. In particular, while a juvenile cancer cell of early cancer secretes only a small amount of extracellular vesicles, and such extracellular vesicles have a low concentration in blood, the above arrangement allows even such extracellular vesicles to be collected efficiently.

The "one or more kinds of lectins being each capable of binding specifically to a surface sugar chain included in an extracellular vesicle derived from a cancer cell" refers to one or more kinds of high-mannose sugar chain binding lectins in a case where the surface sugar chain is a high-mannose sugar chain. In other words, only one kind of high-mannose sugar chain binding lectin may be immobilized on an immobilization support, or two or more kinds of high-mannose sugar chain binding lectins may be immobilized on an immobilization support. In a case where two or more kinds of high-mannose sugar chain binding lectins are used, the two or more kinds of high-mannose sugar chain binding lectins may be used at any amount ratio.

In a case where the surface sugar chain is a sialyl Lewis sugar chain, the one or more kinds of lectins are one or more kinds of sialyl Lewis sugar chain binding lectins. In a case where two or more kinds of sialyl Lewis sugar chain binding lectins are used, the two or more kinds of sialyl Lewis sugar chain binding lectins may be used at any amount ratio.

In a case where the surface sugar chain is a core α1-6 fucose, the one or more kinds of lectins are one or more kinds of core α1-6 fucose binding lectins. In a case where two or more kinds of core α1-6 fucose binding lectins are used, the two or more kinds of core α1-6 fucose binding lectins may be used at any amount ratio. However, Hypnin A is used preferably in a large amount as described later.

The expression "includes one or more immobilization supports...corresponding respectively to one or more kinds of the surface sugar chain" above means including an immobilization support corresponding to each kind of surface sugar chain included in an extracellular vesicle as a capture target.

In a case where, for instance, only an extracellular vesicle including a high-mannose sugar chain binding lectin is to be captured, the extracellular vesicle capturing section simply includes an immobilization support on which one or more kinds of high-mannose sugar chain binding lectins are immobilized. In a case where, for instance, (i) an extracellular vesicle including a high-mannose sugar chain, (ii) an extracellular vesicle including a sialyl Lewis sugar chain, and (iii) an extracellular vesicle including a core α1-6 fucose are to be captured, the extracellular vesicle capturing section includes (i) an immobilization support on which one or more kinds of high-mannose sugar chain binding lectins are immobilized, (ii) an immobilization support on which one or more kinds of sialyl Lewis sugar chain binding lectins are immobilized, and (iii) an immobilization support on which one or more kinds of core α1-6 fucose binding lectins are immobilized.

In a case where the extracellular vesicle capturing section includes immobilization supports on which lectins are immobilized that correspond to as many kinds as possible of surface sugar chains that may be included in extracellular vesicles which may be included in a sample such as blood, the cancer diagnosis device can carry out diagnosis of more kinds of cancer. The extracellular vesicle capturing section thus preferably includes immobilization supports on which lectins are immobilized that correspond respectively to a high-mannose sugar chain, a sialyl Lewis sugar chain, and a core α1-6 fucose.

As used herein, the term "sugar chain" refers to a linear-chain or branched oligosaccharide or polysaccharide. An oligosaccharide results from dehydration binding of 2 to 10 monosaccharides or derivative substitutions thereof. A polysaccharide is a carbohydrate including even more monosaccharides bound together.

The "high-mannose sugar chain" is a sugar chain that has a common scaffold structure made of [Manα1-6(Manα1-3)Manβ1-4GlcNAc] called "trimannosyl core", which is common to N-type sugar chains, and that has only an α-mannose residue in its branch-structure portion. The "high-mannose sugar chain" includes the heptasaccharide [Manα1-6(Manα1-3)Manα1-6(Manα1-3)Manβ1-4GlcNAcβ1-4G 1cNAc] as a common scaffold.

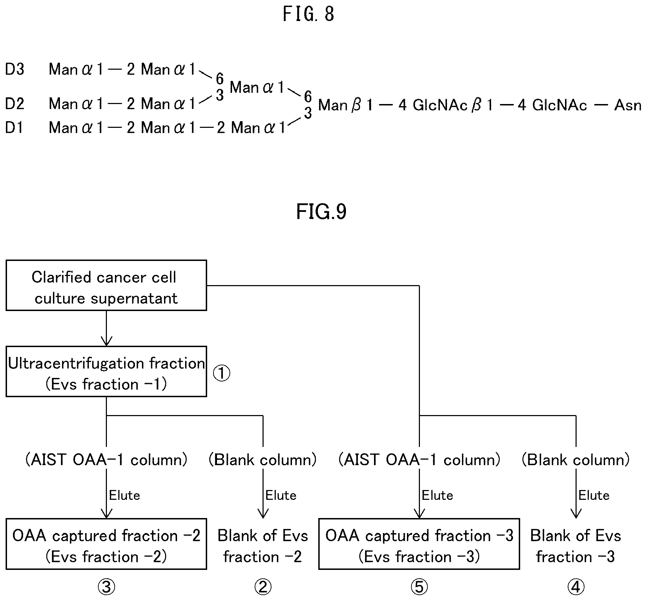

The high-mannose sugar chain binding lectin capable of binding specifically to a high-mannose sugar chain is, for example, one or more kinds of lectins selected from the group consisting of type-I lectin, type-II lectin, type-Ill lectin, and type-IV lectin, the four types being classified on the basis of the difference in the recognition site for a branched oligomannoside and the primary structure (see Kanji Hori, Bioscience & Industry, vol. 71 No. 2 (2013) 129-133). Fig. 8 shows an example structure of a high-mannose sugar chain. In the drawing, D1 to D3 represent D1 arm to D3 arm, respectively.
(a) Type I: Strongly binds to a nonreducing terminal of a D2 arm of a high-mannose sugar chain which terminal has an α(1-3)Man residue. Has a significantly decreased binding force with respect to a residue similar to the above but having α(1-2)Man added. Recognizes a Manα1-6(Manα1-3)Manα1-6(Manα1-3)-Man structure.
(b) Type II: Recognizes (i) an α(1-2)Man residue at a nonreducing terminal of a D1 arm, (ii) an α(1-2)Man residue at a nonreducing terminal of a D2 arm, and (iii) an α(1-2)Man residue at a nonreducing terminal of a D3 arm. Binds more strongly to a high-mannose sugar chain having more α(12)Man residues. Does not bind to a high-mannose sugar chain having no α(1-2)Man residue at a nonreducing terminal.
(c) Type III: Does not recognize a difference in the structure of a branched sugar chain portion, and binds to any high-mannose sugar chain and free trimannosyl core structure
   ((Manα1-6(Manα1-3)Manβ1-4GlcNAcβ1-4GlcNAc-PA). Tends to bind more to a high-mannose sugar chain than to a free trimannosyl core structure.
(d) Type IV: Binds only to a nonreducing terminal of a D3 arm which terminal has an α(1-2)Man residue.

Examples of a lectin belonging to any of (a) to (d) above include algae-derived lectins, which can be isolated from algae or blue-green algae. The word "lectin" is a generic name of a protein that includes molecules having a sugar-binding domain and that is not an antibody.

Examples of the algae-derived lectin include, as type-I lectins, OAA (UniProtKB/ Swiss-Prot Accession No.: P84330) derived from freshwater blue-green algae Oscillatoria agardhii; KAA (KAA-1 (GenBank Accession No: LC007080, also known as ECA-1), KAA-2 (GenBank Accession No: LC007081, also known as ECA-2), KAA-3) derived from algae Kappaphycus alvarezii; KSA (KSA-1, KSA-2) derived from algae Kappaphycus striatum; ESA (ESA-1, ESA-2 (GenBank Accession No.: P84331) derived from algae Eucheuma serra; EAA (EAA-1, EAA-2, EAA-3) derived from algae Eucheuma amakusaensis; EDA (EDA-1, EDA-2 (GenBank Accession No.: LC007085), EDA-3) derived from algae Eucheuma denticulatum; Solnin (Solnin A, Solnin B, Solnin C) derived from algae Solieria pacifica; MPA (MPA-1 (GenBank Accession No: LC008514), MPA-2 (GenBank Accession No: LC008515)) derived from algae Meristotheca papulosa; Granin-BP derived from algae Gracilaria bursa-pastoris; ASL (ASL-1 (GenBank Accession No: LC007083), ASL-2 (GenBank Accession No: LC007084)) derived from algae Agardhiella subulata; PFL (GenBank Accession No: ABA72252) derived from bacteria Pseudomonas fluorescens; MBHA (GenBank Accession No: M13831 derived from bacteria Myxococcus xanthus; and BOA (GenBank Accession No: AIO69853) derived from bacteria Burkholderia oklahomensis.

Examples of the type-II lectin include BCA (GenBank Accession No: BAK23238) derived from algae Boodlea coacta.

Examples of the type-Ill lectin include BPL17 (GenBank Accession No: BAI43482) derived from algae Bryopsis plumosa; BCL17 (GenBank Accession No: LC008516) derived from Bryopsis corticulans; and BML17 (GenBank Accession No: BAI94585) derived from algae Bryopsis maxima.

Examples of the type-IV lectin include MPL-1 (GenBank Accession No: LC007082), MPL-P2, MPL-2, and MPL-P4 derived from algae Meristhotheca papulosa.

A sialyl Lewis sugar chain is a sugar chain that includes sialic acid (N-acetylneuramic acid), galactose, N-acetylglucosamine, and fucose and that is bound to a protein.

Examples of the sialyl Lewis sugar chain binding lectin (that is, a lectin that binds to a sialyl Lewis sugar chain) include a selectin (E-selectin, L-selectin, P-selectin), which is a Ca²⁺-dependent, animal-derived type-C lectin. Only one kind of sialyl Lewis sugar chain binding lectin may be used, or two or more kinds of sialyl Lewis sugar chain binding lectins may be used.

A selectin binds to a sialyl Lewis sugar chain and can be used as a sialyl Lewis sugar chain binding lectin.

Examples of the core α1-6 fucose binding lectin (that is, a lectin that binds to a core α1-6 fucose) include Hypnin A (Hypnin A-1 (Accession No: JC5773), Hypnin A-2 (Accession No: JC5774), Hypnin A-3 (Accession No: P85888)) derived from Hypnea japonica; Hc-hypnin-A (Hc-hypnin A-1 (GenBank Accession No: LC013892), Hc-hypnin A-2, Hc-hypnin A-3) derived from algae Hypnea cervicornis; LCA (GenBank Accession No: P02870) derived from Lens culinaris; AAL (GenBank Accession No: P18891) derived from Aleuria aurantia; AOL (GenBank Accession No: BAB88318) derived from Aspergillus oryzae; and PhoSL (GenBank Accession No: LF715849) derived from Pholiota squarrosa.

Hypnin A, among other core α1-6 fucose binding lectins, has high specificity in binding to a core α1-6 fucose. The core α1-6 fucose binding lectin is thus preferably Hypnin A. In a case where two or more kinds of core α1-6 fucose binding lectins are used, Hypnin A is used preferably in an amount larger than the amount of any other lectin used. The use of Hypnin A allows for further increased specificity in binding to a core α1-6 fucose included in an extracellular vesicle derived from a cancer cell, and can thereby increase the specificity in cancer diagnosis.

Hypnin A may be any of Hypnin A-1, Hypnin A-2, and Hypnin A-3 mentioned above. It is possible to use, as Hypnin A, two or more kinds of Hypnin A selected from the group consisting of Hypnin A-1, Hypnin A-2, and Hypnin A-3. In a case where two or more kinds of Hypnin A are used, the two or more kinds of Hypnin A may be mixed at any ratio.

AOL, AAL, and LCA each have specificity in binding to a core α1-6 fucose which specificity is lower than that of Hypnin A, but does bind sufficiently to a core α1-6 fucose. AOL, AAL, and LCA can thus be used as a core α1-6 fucose binding lectin.

Whether the lectin has a property of binding to a sugar chain, that is, whether the lectin binds to a sugar chain, can be determined by, for example, (i) passing the lectin as a test subject through a column on which a sugar chain or an antibody, glycoprotein, or the like to which a sugar chain is bound is immobilized as a target and (ii) evaluating, on the basis of the amount of lectin included in the passed liquid or the amount of lectin eluted from the column with use of a specific eluent, whether the lectin has been bound to the column.

Whether the lectin has a property of binding to a sugar chain, that is, whether the lectin binds to a sugar chain, can be evaluated by (i) Western blot (see The Research and Practice in Forensic Medicine, 37, 155, 1994), which involves immobilizing on a membrane or the like an antibody to which a sugar chain as a target is bound and detecting the antibody with use of a polypeptide labeled with, for example, biotin, fluorescein isothiocyanate, or peroxidase or (ii) dot blot method (see Analytical Biochemistry, 204(1), 198, 1992).

Alternatively, surface plasmon resonance (SPR) method may be used to measure the affinity between (i) a chip on which a sugar chain or an antibody, glycoprotein, or the like to which a sugar chain is bound is immobilized as a target and (ii) a lectin as a test subject. The above method is preferable because it allows not only the presence or absence of affinity but also the strength thereof to be measured. In a case where the affinity constant (K_{A}) obtained during the measurement is not less than 10 (M⁻¹), more preferably not less than 10³ (M⁻¹), most preferably not less than 10⁴ (M⁻¹), the lectin and the sugar chain can be determined as being bound to each other.

Those lectins mentioned above as examples which are other than PFL, MBHA, BOA, selectin, LCA, AAL, AOL, and PhoSL can be isolated from algae or blue-green algae by a conventional method.

For instance: ESA-1 can be isolated by a method disclosed in Kawakubo, A. et al., J. Appl. Phycol. 9, 331-338, 1997; EAA-1, EAA-2, and EAA-3 can be isolated by a method disclosed in Kawakubo, A. et al., J. Appl. Phycol. 11, 149-156, 1999; EDA-1 and EDA-3 can be isolated by a method disclosed in Hung, L. D. et al., J. Appl. Phycol. 27, 1657-1669, 2015; KAA-3 can be isolated by a method disclosed in Hung, L. D. et al., Fish. Sci. 75, 723-730, 2009; KSA-1 and KSA-2 can be isolated by a method disclosed in Hung, L. D. et al., Phytochemistry 72, 855-861, 2011; Solnin A, Solnin B, and Solnin C can be isolated by a method disclosed in Hori, K. et al., Phytochemistry 27, 2063-2067, 1988; Granin-BP can be isolated by a method disclosed in Okamoto, T. et al., Experientia. 46, 975-977, 1990; and MBHA can be isolated by a method disclosed in Cumsky, M. G., Zusman, D. R., J. Biol. Chem. 256, 12581-12588, 1981.

The lectin may be produced (i) by refining a natural substance, (ii) through a chemical synthesis procedure, or (iii) from a prokaryote or eukaryote host (including, for example, a bacterial cell, a yeast cell, a higher plant cell, an insect cell, and a mammalian cell) with use of recombination technique. These lectins may be commercially available products.

In an embodiment, the above lectins may each be a polypeptide having a publicly disclosed amino acid sequence or a variant thereof.

Examples of the variant include variants including a deletion, an insertion, an inversion, a repetition, and a type substitution (for example, substitution of a hydrophilic residue with another residue).

It is well-known in the related technical field that some amino acids in an amino acid sequence of a polypeptide can easily be modified without significantly affecting the structure or function of the polypeptide. Further, apart from the artificial modification, it is also known that there exists, in a natural protein, a variant which does not cause a significant change in the structure or function of the protein. A person skilled in the art can easily modify one or more amino acids of an amino acid sequence of a polypeptide with use of a well-known technique.

A preferable variant has a conservative or non-conservative amino acid substitution, deletion, or insertion. A variant preferably has a silent substitution, insertion, or deletion. A variant particularly preferably has a conservative substitution. These do not change the polypeptide activity for an embodiment of the present invention.

Substitutions regarded representatively as conservative substitutions are (i) substitution of one of aliphatic amino acids Ala, Val, Leu, and lie with another amino acid, (ii) exchange of hydroxyl residues Ser and Thr, (iii) exchange of acidic residues Asp and Glu, (iv) substitution between amide residues Asn and Gln, (v) exchange of basic residues Lys and Arg, and (vi) substitution between aromatic residues Phe and Tyr.

The lectins are each preferably (i) a polypeptide including a publicly disclosed amino acid sequence or (ii) a polypeptide including an amino acid sequence identical to the above amino acid sequence except that one or more amino acids have been substituted, deleted, inserted, or added.

The expression "one or more amino acids have been substituted, deleted, inserted, or added" above means that one or more amino acids have been substituted, deleted, inserted, or added in a number that can be substituted, deleted, inserted, or added by a publicly known mutant polypeptide preparation method such as site-directed mutagenesis. The number is preferably not more than 10, more preferably not more than 7, most preferably not more than 5. Such a mutant polypeptide is not limited to a polypeptide having a mutation artificially introduced by a publicly known method for preparing a mutant polypeptide as described above, and may be a polypeptide produced by isolating and purifying a naturally occurring polypeptide.

The lectin for use in a cancer diagnosis device in accordance with an embodiment of the present invention is preferably immobilized on an immobilization support through orientation control. The lectin is, in other words, preferably immobilized on an immobilization support at one end of a peptide chain. This point will be described later. In order to control the orientation to immobilize the lectin on an immobilization support, it is more preferable that (1) a naturally derived amino acid sequence of the lectin have not more than one cysteine residue, (2) a naturally derived amino acid sequence of the lectin be free from a cysteine residue, or (3) a naturally derived amino acid sequence of the lectin be free from a cysteine residue or a lysine residue. Thus, while it is possible to carry out the amino acid substitution or amino acid addition described above, it is preferable not to substitute an amino acid with cysteine or lysine.

The lectin for use in a cancer diagnosis device in accordance with an embodiment of the present invention may be a polypeptide including peptide-bonded amino acids, but is not limited to that. The lectin may be a complex polypeptide including a structure other than a polypeptide. As used herein, the "structure other than a polypeptide" is, for example, a sugar chain or an isoprenoid group, but is not limited to any particular structure.

The lectin may include an additional polypeptide. Examples of the additional include epitope-tagged polypeptides such as His, Myc, and Flag.

The lectin may be expressed as a recombinant in a modified form such as fusion protein. As discussed later, in a case where the immobilization support is a monolithic gel such as a silica monolith, the lectin is preferably expressed as a recombinant resulting from adding (i) a linker sequence, (ii) an immobilization reaction sequence, and (iii) a purification tag sequence to the carboxy terminus of the polypeptide. The purification tag sequence can contribute to simple purification of a fusion protein, and be removed before final preparation of a polypeptide.

Examples of the purification tag sequence include (i) a hexahistidine peptide (for example, a tag provided in the pQE vector (Qiagen, Inc.)) and (ii) an "HA" tag useful for purification corresponding to an epitope derived from an influenza hemagglutinin (HA) protein.

### (B) Immobilization support on which the lectin is immobilized

A cancer diagnosis device in accordance with an embodiment of the present invention includes an immobilization support on which the lectin is immobilized. Examples of the immobilization support include (i) an inorganic immobilization support such as monolithic gel and beads (for example, glass beads), (ii) latex or beads made of a natural or synthesized polymer, and (iii) an organic immobilization support (filter) such as fiber, woven fabric, nonwoven fabric, and hollow fiber. These immobilization supports are each preferably an immobilization support having a primary amino group to facilitate the later-described control of the orientation.

The beads are preferably, for example, lectin-solid-phased magnetic beads in which (i) a complex of a lectin tagged with histidine or the like and an antibody against the tag and (ii) magnetic beads in which protein G is solid-phased are bound to each other. The lectin-solid-phased magnetic beads can be prepared by causing the antibody in the complex to react with the protein G.

Examples of commercially available immobilization supports each having a primary amino group include amino-cellulofine (product name: available from Seikagaku Corporation), AF-Amino Toyopearl (product name: available from TOSOH), EAH-Sepharose 4B and lysine-Sepharose 4B (product names: available from Amersham Pharmacia), and Porus 20NH (product name: available from Boehringer Mannheim). The immobilization support having a primary amino group may be prepared by introducing into glass beads a primary amino group with use of a silane compound having a primary amino group (for example, 3-aminopropylmethoxysilane) .

The immobilization support is, among others, preferably a monolithic gel. A monolithic gel has (i) macropores serving as a solution flow path and (ii) mesopores serving as a separation space, and thus allows the lectin to be immobilized thereon easily. A monolithic gel can also process whole blood. A monolithic gel thus allows for efficient binding between the lectin and a surface sugar chain included in an extracellular vesicle in an analyte.

A monolithic gel is a gel made of a monolithic material (monolithic polymer). A monolithic material is made of a single, continuous structure and has pores each serving as a continuous flow path extending through the structure. A monolithic gel can be produced through (i) a solating step of preparing an aqueous solution sol, (ii) a gelating step of heating the resulting sol into a gel, and (iii) a firing step of firing the resulting gel.

A monolithic gel can be produced by, for example, subjecting a reaction solution containing silica as a main component to sol-gel transition involving phase separation. A precursor of a network component for use in the sol-gel reaction for causing gel formation is a metal alkoxide, a complex, a metal salt, an organically modified metal alkoxide, an organically crosslinked metal alkoxide, and a multimer as a partially hydrolyzed or partially polymerized product thereof. It is also possible to similarly use sol-gel transition caused by changing the pH of water glass or an aqueous silicate solution.

More specifically, the monolithic gel is preferably produced by (i) dissolving a water-soluble polymer and a pyrolytic compound in an acidic aqueous solution, (ii) adding, to the resulting solution, a metal compound with a hydrolytic functional group for a hydrolysis reaction, (iii) after the resulting product is solidified, heating a wet gel for pyrolysis of a low molecular weight compound dissolved in the gel in advance during preparation of the gel, and (iv) drying and heating the resulting product.

The water-soluble polymer is a water-soluble organic polymer that is theoretically dissolvable in an aqueous solution to have an appropriate concentration and that is uniformly dissolvable in a reaction system including an alcohol produced by the metal compound having a hydrolytic functional group. Specifically, suitable examples of the water-soluble polymer include (i) a sodium salt or potassium salt of polystyrene sulfonate as a polymeric metal salt, (ii) polyacrylic acid, as a polymer acid, that is dissociated into a polyanion, (iii) polyallyl amine or polyethylene imine, as a polymeric base, that generates a polycation in an aqueous solution, (iv) polyethylene oxide, as a neutral polymer, that has an ether bond in the main chain, and (v) polyvinylpyrrolidone that has a carbonyl group in a side chain. The organic polymer may be replaced with formamide, a polyvalent alcohol, or a surfactant. In this case, the polyvalent alcohol is suitably glycerine, and the surfactant is suitably a polyoxyethylene alkyl ether.

The metal compound having a hydrolytic functional group is a metal alkoxide or an oligomer thereof. These preferably have a small number of carbon atoms such as a methoxy group, an ethoxy group, and a propoxy group. The metal is a metal of a finally produced oxide, for example, Si, Ti, Zr, or Al. Only one kind of the metal may be used, or two or more kinds of the metal may be used. The oligomer is an oligomer that can be dissolved and dispersed uniformly in an alcohol. The oligomer can, specifically, be up to about a decamer.

The acidic aqueous solution normally contains a mineral acid such as hydrochloric acid and nitric acid at a molar concentration of not less than 0.001 or preferably contains an organic acid such as acetic acid and formic acid at a molar concentration of not less than 0.01.

The phase separation and gelation can be achieved by keeping the solution in a room with a temperature of 40°C to 80°C for 0.5 to 5 hours. The phase separation and gelation occur as the initially transparent solution becomes whitish, the silica phase and the aqueous phase become separated from each other, and the solution becomes gelated. The phase separation and gelation cause the water-soluble polymer to be dispersed, so that the water-soluble polymer is substantially not precipitated.

Specific examples of the pyrolytic compound dissolved together in advance include organic amides such as urea or hexamethylene tetramine, (ii) formamide, (iii) N-methylformamide, (iv) N,N-dimethylformamide, (v) acetamide, (vi) N-methylacetamide, and (vii) N,N-dimethylacetamide. The pyrolytic compound is, however, not limited to any particular one as long as the pyrolytic compound renders the solvent basic after pyrolysis, because the important condition is the pH value of the solvent after heating.

The pyrolytic compound dissolved together in advance is used in an amount that depends on the kind of the compound. In a case where the pyrolytic compound is, for example, urea, the pyrolytic compound is used in an amount of 0.05 g to 0.8 g, preferably 0.1 g to 0.7 g, with respect to 10 g of the reaction solution. In the case where the pyrolytic compound is, for example, urea, the heating temperature is 40°C to 200°C, and the pH value of the solvent after heating is preferably 6.0 to 12.0.

The pyrolytic compound may alternatively be a pyrolytic compound that produces a compound that dissolves silica through pyrolysis similarly to hydrofluoric acid.

With the above method, a water-soluble polymer is dissolved in an acidic aqueous solution, and a metal compound with a hydrolytic functional group is added to the resulting solution for a hydrolysis reaction. This produces a gel including a solvent-rich phase and a skeleton phase that are separated from each other. After the product (gel) is solidified, the product is matured for an appropriate time period. Then, the wet gel is heated. This causes pyrolysis of the amide-based compound dissolved in advance in the reaction solution, thereby increasing the pH of that portion of the solvent which is in contact with the inner wall of the skeleton phase. The solvent then erodes the inner wall and changes the unevenness of the inner wall to gradually increase the pore diameters.

In a case where the gel contains silica as a main component, the change has a very small degree in an acidic or neutral region. However, as the pyrolysis becomes accelerated, and the aqueous solution becomes more basic, that portion which has pores is dissolved for reprecipitation at a more flat portion. This causes a reaction that increases the average pore diameter to occur significantly.

A gel that is free from large holes and that only has three-dimensionally constrained pores has a portion that is dissolvable under an equilibrium condition but that has an elution substance which is not diffused even in an external solution. This causes a large proportion of the original pore structure to be left. On the other hand, a gel that has a solvent-rich phase which is to serve as large holes has many pores that are constrained only two-dimensionally, and allows substances to be exchanged sufficiently frequently with an external aqueous solution. Thus, as large pores develop, small pores disappear. The overall pore diameter distribution is not spread significantly.

The heating process is effectively carried out in a case where the gel is hermetically sealed, and the vapor pressure of the pyrolysis product is saturated so that the pH of the solvent rapidly reaches a steady-state value.

The time period of the heat treatment which time period is necessary for (i) the dissolution and reprecipitation reaction to be in a steady state and (ii) a corresponding pore structure to be obtained depends on the size of the large holes and the volume of the sample. It is thus necessary to select a minimum processing time period that does not substantially change the pore structure under each processing condition.

After the gel undergoes the heat treatment, the solvent is vaporized, so that a dried gel closely adheres to the tube wall in the groove. This dried gel may still contain the coexisting substances in the starting solution. The gel is thus heat-treated at an appropriate temperature for pyrolysis of an organic substance and the like. This allows a desired inorganic porous material to be obtained. The drying operation is carried out by letting the gel stand at 30°C to 80°C for several hours to several tens of hours. The heat treatment is carried out at approximately 200°C to 800°C.

Examples of the monolithic gel include (i) organic monoliths such as an acrylamide-based monolith, a methacrylic acid ester-based monolith, and a styrene-divinylbenzene-based monolith and (ii) a silica monolith. The monolith gel is particularly preferably a silica monolith because a silica monolith allows a primary amino group described later to be introduced easily. The monolith gel is, however, not limited to that. The monolithic gel is produced by, for example, a method disclosed in Japanese Examined Patent Publication, *Tokukohei,* No. 08-029952 or Japanese Patent Application Publication, *Tokukaihei,* No. 07-041374. A silicon-containing silica monolith is produced by mixing acetic acid, polyethylene glycol, and tetramethoxysilane with one another and firing the mixed solution at, for example, 40°C for 24 hours.

The monolithic gel preferably has a macropore diameter of not less than 1 µm and not more than 200 µm and a mesopore diameter of not less than 10 nm and not more than 300 nm.

With the above arrangement, the macropore diameter has a size that allows blood cells to pass through, and the mesopore diameter has a size that allows extracellular vesicles such as an exosome and a microparticle to pass through. Thus, immobilizing the lectin on the monolithic gel allows a surface sugar chain included in an extracellular vesicle derived from a cancer cell and the lectin to be bound specifically to each other. The above arrangement thereby allows an extracellular vesicle to be separated efficiently from an analyte.

The monolithic gel more preferably has a macropore diameter of not less than 20 µm and not more than 200 µm and a mesopore diameter of not less than 40 nm and not more than 200 nm. This arrangement allows blood cells to pass through while reducing the load pressure on the macropores, and also allows a high separation capability to be maintained. With the above arrangement, the mesopores allow extracellular vesicles such as an exosome and a microparticle to pass through more easily, and keep high the amount of extracellular vesicles that are separable per unit volume. The above arrangement thus makes it possible to (i) efficiently separate, from an analyte, a protein including a surface sugar chain which protein is expressed in an extracellular vesicle and also (ii) easily process an analyte such as whole blood.

The monolithic gel can be prepared by sol-gel method as described above. A monolithic gel having a desired macropore diameter such as not less than 1 µm and not more than 200 µm or not less than 20 µm and not more than 200 µm can be produced by (i) adjusting the molecular weight and amount of a water-soluble polymer to be added to induce phase separation and the amount of block copolymer that influences when phase separation occurs and (ii) adjusting the viscosity of the sol.

The mesopore diameter of the monolithic gel can be controlled on the basis of the aging condition applied after the gelation. For instance, adjusting the solvent composition, heating temperature, and heating time period for the aging allows a monolithic gel to be produced that has a desired mesopore diameter such as not less than 10 nm and not more than 300 nm or not less than 40 nm and not more than 200 nm.

The macropore diameter and the mesopore diameter can be determined by, for example, (i) measuring the distribution of pore diameters by a conventionally publicly known method such as mercury press-in method or (ii) observing the monolithic gel under a microscope.

The monolithic gel may be a commercially available product. Examples of the commercially available product include a MonoBis column (low-pressure type, unmodified, product number: 3250L30SI, macropore diameter: 1.4 µm, mesopore diameter: 30 nm, available from Kyoto Monotech Co., Ltd.).

The lectin is immobilized preferably in an amount of not less than 2 µg, more preferably not less than 10 µg, even more preferably not less than 100 µg, particularly preferably not less than 1 mg, with respect to 1 ml of the immobilization support. With this arrangement, the lectin is immobilized at a high density per unit volume of the immobilization support. This is preferable because such a high density makes it possible to efficiently separate from an analyte an extracellular vesicle including a surface sugar chain that binds to the lectin.

The amount of lectin immobilized with respect to 1 ml of the immobilization support has an upper limit of preferably not more than 10 mg.

Whether the lectin is immobilized on the immobilization support in a desired amount can be determined by, for example, (i) measuring the absorbance at 280 nm of lectin in the reaction solution before and after the reaction, (ii) on the basis of the measurement result, determining the amount of lectin consumed for the reaction, and (iii) regarding the amount as an immobilized amount.

### (C) Method for immobilizing the lectin on the immobilization support

The lectin may be immobilized on the immobilization support by any method. It is, however, preferable to control the orientation of the lectin and immobilize the lectin on the immobilization support in order to immobilize the lectin at a high density as described above. Controlling the orientation of the lectin and immobilizing the lectin means immobilizing the lectin on the immobilization support at one end of a peptide chain, for example, immobilizing the lectin at the carboxy terminus of the peptide chain. Controlling the orientation of the lectin and immobilizing the lectin can be carried out by, for example, a method disclosed in Japanese Patent No. 2517861, Japanese Patent Application Publication, *Tokukai,* No. 2000-119300, or Japanese Patent Application Publication, *Tokukai,* No. 2003-344396.

In a case where the immobilization support is a monolithic gel, it is preferable that for immobilization of the lectin, (i) an epoxy group be introduced into the monolithic gel, and (ii) an amino group-containing polymer be then introduced for introduction of a primary amino group into the monolithic gel at a high density. This allows the orientation of the lectin to be controlled and the lectin to be immobilized on the monolithic gel efficiently.

An epoxy group can be introduced into the monolithic gel by, for example, immersing the monolithic gel into an epoxysilane solution. A primary amino group can be introduced by adding an amino group-containing polymer to a surface of the monolithic gel for permeation through the monolithic gel. These operations allow an epoxy group to be introduced into the monolithic gel and a primary amino group to be covalently bonded to the epoxy group.

As an example of how the lectin is immobilized on the primary amino group, the description below deals with a method based on a method disclosed in Japanese Patent Application Publication, *Tokukai,* No. 2000-119300.

The lectin (polypeptide) is represented by General Formula (2).

NH₂-R₁-COOH ... (2)

In the formula, R₁ represents an amino acid residue.

Next, the polypeptide represented by General Formula (2) is bound to a peptide represented by General Formula (3) below to prepare a fusion polypeptide represented by General Formula (4) below. The SH group of the fusion polypeptide is cyanated so that the fusion polypeptide is converted into a cyano group-containing polypeptide represented by General Formula (5) below. This cyano group-containing polypeptide is bound to a primary amino group introduced into the immobilization support represented by General Formula (6) below. This allows the lectin to be immobilized on the immobilization support as a polypeptide represented by General Formula (1) below.

NH₂-R₂-CO-NH-CH(CH₂-SH)-CO-X ... (3)

In the formula, R₂ represents an amino acid residue, and X represents OH or an amino acid residue.

NH₂-R₁-CO-NH-R₂-CO-NH-CH(CH₂-SH)-CO-X ... (4)

In the formula, R₁ and R₂ each represent an amino acid residue, and X represents OH or an amino acid residue.

NH₂-R₁-CO-NH-R₂-CO-NH-CH(CH₂-SCN)-CO-X ... (5)

In the formula, R₁ and R₂ each represent an amino acid residue, and X represents OH or an amino acid residue.

NH₂-Y ... (6)

In the formula, Y represents an immobilization support.

NH₂-R₁-CO-NH-R₂-CO-NH-Y ... (1)

In the formula, R₁ and R₂ each represent an amino acid residue, and Y represents an immobilization support.

As shown in General Formula (1), R₂ serves as a linker peptide (linker sequence) between (i) the polypeptide represented by General Formula (2) to be immobilized and (ii) a primary amino group. R₂ is an amino acid residue, whose kind and number are not particularly limited. R₂ is, for example, a sequence of five glycine residues or six glycine residue. R₁ is also an amino acid residue, whose kind and number are not particularly limited.

In the General Formula (3), the portion -NH-CH(CH₂-SH)-CO-X is referred to herein as an immobilization reaction sequence. This portion is cyanated as shown in the General Formula (5) to produce a cyano group-containing polypeptide. This allows reaction to occur between the above portion and the immobilization support represented by the General Formula (6).

The immobilization reaction sequence requires a single cysteine residue as shown in the General Formula (3). X represents OH or an amino acid residue (whose kind and number are not particularly limited). X is not particularly limited. Each substance shown in the General Formula (4) preferably has an isoelectric point of 4 to 5. X is thus preferably a sequence containing a large amount of aspartic acid or glutamic acid because such a sequence allows the isoelectric point to be adjusted to 4 to 5 easily. X is, for example, a sequence including six aspartic acid residues or alanyl-polyaspartic acid. Alanyl-polyaspartic acid tends to allow an amide bond forming reaction to occur via a cyanocysteine residue by causing the amino acid at the next position of cyanocysteine represented by the General Formula (5) to be alanine. Further, it is easy to adjust the isoelectric point to 4 to 5 because the carboxyl group of aspartic acid is the most acidic in the amino acid side chain.

The immobilization reaction sequence is not limited to any particular sequence as long as the immobilization reaction sequence includes a single cysteine residue. The immobilization reaction sequence is, for example, a sequence including eight amino acid residues including a single cysteine residue. An example of the sequence is disclosed in Patent Literature 2. X above is preferably such that the purification tag sequence mentioned above is also added to the C-terminus side of the immobilization reaction sequence.

The polypeptide represented by the General Formula (2) can be converted into the fusion polypeptide represented by the General Formula (4) by a conventionally publicly known recombinant DNA technique. Specifically, a polynucleotide encoding the polypeptide represented by the General Formula (2) and a polynucleotide encoding the peptide sequence represented by the General Formula (3) are bonded to each other. This prepares a polynucleotide encoding the fusion polypeptide represented by the General Formula (4). This is expressed in a host organism such as Escherichia coli. The polypeptide thus expressed is then separated and purified. This allows the intended fusion polypeptide to be prepared.

The fusion polypeptide represented by the General Formula (4) can be converted into the fusion polypeptide represented by the General Formula (5) (that is, cyanation reaction) with use of a cyanation reagent. The cyanation reagent is normally (i) 2-nitro-5-thiocyanobennzoic acid (NTCB) (described in Y. Degani, A. Ptchornik, Biochemistry, 13, 1-11 (1974)) or 1-cyano-4-dimethylaminopyridinium tetrafluoroborate (CDAP) for a simple method.

The NTCB and the CDAP may each be a commercially available product itself. Cyanation involving use of NTCB is advantageous in that cyanation can be carried out efficiently at a pH of 7 to 9 and that the reaction efficiency can be studied on the basis of an increase (molecular extinction coefficient = 13,600 M⁻¹cm⁻¹) in the absorbance at 412 nm of liberated thionitrobenzoic acid. Cyanation of an SH group can also be carried out by, for example, a method disclosed in J. Wood & Catsipoolas, J. Biol. Chem. 233, 2887 (1963).

The reaction between the cyanated fusion polypeptide represented by the General Formula (5) and the immobilization support represented by the General Formula (6) can be carried out under a weak alkaline condition (pH of 8 to 10) at room temperature.

The immobilization reaction may involve use of any solvent that allows the cyanated fusion polypeptide represented by the General Formula (5) to be dissolved therein and that has an adjustable pH. Examples of the solvent include (i) various buffer solutions such as a phosphate buffer and a borate buffer, (ii) alcohols such as methanol and ethanol, (iii) dimethylformamide, and (iv) dimethyl sulfoxide. Regarding the reaction temperature, room temperature allows for a high reaction efficiency. The reaction temperature may be any temperature within a range within which a solvent used does not freeze or boil and within which the cyanated fusion polypeptide represented by the General Formula (5) is not denatured to aggregate.

In a case where a lectin that binds specifically to the surface sugar chain is immobilized on an immobilization support as described above, the lectin is immobilized on the immobilization support at the carboxy terminus of the peptide chain. Specifically, since the orientation of the lectin is controlled, and the lectin is immobilized on the immobilization support, the lectin is immobilized on the immobilization support at a high density. This increases the efficiency of binding of the surface sugar chain, and thereby allows an extracellular vesicle included in an analyte to be separated from the analyte more efficiently. The above arrangement prevents immobilized peptides from colliding with each other, and thereby allows the polypeptide to be denatured reversibly.

The immobilization support is preferably charged into a column or the like for use so that an analyte can be introduced easily and that the surface sugar chain can be bonded to the lectin smoothly. Examples of the column include a conventionally publicly known spin column and a stainless steel column for liquid chromatography.

The immobilization support may be disposed on a microchip. This arrangement will be described later.

### < 1-2. Extracting microRNA from extracellular vesicle>

The extracellular vesicle capturing section uses a lectin that can bind specifically to a surface sugar chain of a protein included in an extracellular vesicle derived from a cancer cell, and thereby allows, not an extracellular vesicle derived from a normal cell, but the above extracellular vesicle to be captured selectively. Extracting microRNAs from the captured extracellular vesicle and analyzing the pattern of the surface sugar chain and the pattern of how the microRNAs are present allows (i) the kind of an initial cancer to be determined and (ii) recurrence and metastasis to be diagnosed at an initial stage. The description below deals with how to extract a microRNA from the immobilization support.

Washing the immobilization support sufficiently with use of PBS and then treating the immobilization support with use of, for example, a surfactant makes it possible to extract a microRNA contained in an extracellular vesicle bound to the lectin.

Examples of the extracellular vesicle include an exosome and/or a microparticle as described above. The exosome and the microparticle each individually contain a microRNA. Changing the concentration of the surfactant makes it possible to separately extract a microRNA contained in the exosome and a microRNA contained in the microparticle. This is because the microparticle and the exosome have respective degrees of disintegration with respect to the surfactant which degrees differ from each other. Examples of the surfactant include SDS, TritonX-100, deoxycholic acid, and Tween20.

The immobilization support is, for instance, first treated with a low-concentration surfactant (for example, 0.01% by weight of SDS and 0.025% by weight of TritonX-100) for disintegration of the microparticle so that the contained microRNA can be extracted.

Next, after a microRNA has been collected from the microparticle, the immobilization support is washed sufficiently with, for example, PBS. Then, the immobilization support is treated with a high-concentration surfactant (for example, 0.125% by weight of SDS and 0.075% by weight of TritonX-100). This causes the exosome to be disintegrated, so that the contained microRNA can be extracted. The treatment with a surfactant can be carried out by, for instance, passing a surfactant liquid through a column charged with the immobilization support.

Such extraction is carried out for each of immobilization supports provided in one-to-one correspondence with one or more kinds of surface sugar chains. This allows a microRNA contained in the exosome and a microRNA contained in the microparticle to be separately collected each in correspondence with a surface sugar chain included in an extracellular vesicle. In a case where, for instance, miR-1 is contained in a liquid extracted from an immobilization support for a high-mannose sugar chain binding lectin, the subject can be diagnosed, on the basis of the relationship between a microRNA and a cancer type (for example, Yuqing He et al., Clinical Chemistry, 61:9, 1138-1155, 2015), as being affected with breast cancer. A cancer diagnosis device in accordance with an embodiment of the present invention utilizes the specificity in binding between the surface sugar chain and the lectin to specifically capture an extracellular vesicle derived from a cancer cell. This allows a microRNA derived from a cancer cell to be collected specifically and rapidly. The cancer diagnosis device is thus capable of cancer diagnosis with high specificity.

A cancer diagnosis device in accordance with an embodiment of the present invention preferably includes a microRNA accommodating section for accommodating, in accordance with the kind of surface sugar chain, (i) a microRNA extracted from an exosome and (ii) a microRNA extracted from a microparticle.

The extracted liquid from the immobilization support may, for instance, be delivered directly to a detecting section described later for analysis of a microRNA. However, including a microRNA accommodating section allows a microRNA extracted from an immobilization support to be reliably separated according to the kind.

The microRNA accommodating section, for instance, includes a single plate having depressions for accommodating a microRNA extracted from an exosome, a microRNA extracted from a microparticle, and waste liquid, respectively.

The extracted liquid obtained through the above extraction contains RNA, ncRNA, DNA, and the like as well as a surfactant in addition to a microRNA. Thus, the extracted liquid can, according to need, be purified by a purification method such as Boom method involving use of a chaotropic ion.

### <1-3. Detecting section for detecting microRNA>

A cancer diagnosis device in accordance with an embodiment of the present invention includes a detecting section for detecting a microRNA included in the extracellular vesicle. The detecting section is, for example, (i) an array to which cDNA for a microRNA that may be included in the extracellular vesicle is fixed as a probe or (ii) a well for amplifying a microRNA.

Regarding a surface sugar chain included in an extracellular vesicle derived from a cancer cell, it is known as described above that (i) an extracellular vesicle derived from a breast cancer cell, a prostate cancer cell, and a melanoma cell has a high-mannose sugar chain, (ii) an extracellular vesicle derived from a liver cancer cell has a core α1-6 fucose, and (iii) an extracellular vesicle derived from a pancreas cancer cell, a gastric cancer cell, and a lung cancer cell has a sialyl Lewis sugar chain.

In view of the above, preparing, for example, an array (detecting section) to which cDNA for a microRNA that may be included in the extracellular vesicle is fixed as a probe makes it possible to diagnose the presence or absence of cancer and the type of cancer rapidly and comprehensively. The array may be prepared by a conventionally publicly known method. The detection of a microRNA with use of the array can be carried out by a conventionally publicly known method.

In other words, cDNA is prepared by a normal method from a microRNA contained in the extracted liquid extracted from an extracellular vesicle captured by the extracellular vesicle capturing section. Next, the cDNA is labeled with a fluorescent dye for use as a sample. The sample is placed on the array so that the cDNA and the probe are hybridized with each other. Subsequently, the signal (fluorescence intensity) of each probe DNA (spot) is scanned. The data is then analyzed with use of a computer. The pattern of a microRNA in which fluorescence has been observed makes it possible to diagnose the presence or absence of cancer or the type of cancer.

The probe is preferably placed in each of the areas on the array which areas are separated according to the type of surface sugar chain. For instance, in an area on the array, cDNA is placed for a microRNA included in an extracellular vesicle derived from cells of breast cancer, prostate cancer, and melanoma, which are related to a high-mannose sugar chain. In another area, cDNA is placed for a microRNA included in an extracellular vesicle derived from cells of pancreas cancer, gastric cancer, and lung cancer, which are related to a sialyl Lewis sugar chain. In still another area, cDNA is placed for a microRNA included in an extracellular vesicle derived from a cell of liver cancer, which is related to a core α1-6 fucose.

The above placement allows the type of cancer to be determined rapidly. In a case where, for instance, fluorescence has been observed for a microRNA included in an extracellular vesicle of melanoma, it is possible, due to the separated areas in which different probes are placed, to rapidly and unambiguously determine that the subject is affected with melanoma.

The well for amplifying the microRNA is used to detect, by RT-PCR or LAMP method, a microRNA included in an extracellular vesicle derived from a cancer cell. Specifically, cDNA is prepared in the well by a normal method from a microRNA extracted from an extracellular vesicle, so that an appropriate primer is designed. The cDNA and the primer are used to amplify DNA. The well is used simply for an amplification reaction, and is thus not limited to any particular structure as long as the well allows an amplification reaction to occur.

### <1-4. Example of cancer diagnosis device and cancer diagnosis>

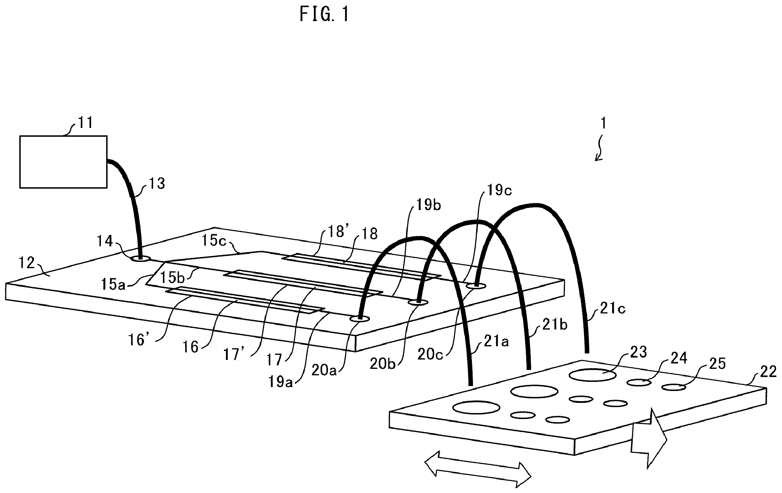

With reference to Figs. 1 to 3, the description below deals with (i) a cancer diagnosis device in accordance with an embodiment of the present invention and (ii) cancer diagnosis involving use of the cancer diagnosis device.

Fig. 1 is a diagram schematically illustrating respective example configurations of the extracellular vesicle capturing section and the microRNA accommodating section, which are included in a cancer diagnosis device in accordance with an embodiment of the present invention. In Fig. 1, 1 represents a cancer diagnosis device, 11 represents a liquid delivery system, 12 represents a plate, 13, 15a to 15c, 19a to 19c, and 21a to 21c represent first to fourth flow paths, 14 represents an introduction section, 16 to 18 represent extracellular vesicle capturing sections, 16' to 18' represent extracellular vesicle capturing section accommodating sections, 20a to 20c represent discharge sections, 22 represents a microRNA accommodating section, 23 represents waste liquid accommodating sections, 24 represents accommodating sections for a microRNA extracted from a microparticle, and 25 represents accommodating sections for a microRNA extracted from an exosome. The detecting section (not shown) is provided adjacently to the microRNA accommodating section in the direction of the arrow. The plate 12 is preferably made of a transparent material such as glass, an acrylic resin material, a cycloolefin resin material, and a polyester resin material.

As illustrated in Fig. 1, a cancer diagnosis device 1 in accordance with an embodiment of the present invention preferably includes an introduction section 14 configured to introduce a sample into the extracellular vesicle capturing sections; one or more discharge sections 20a to 20c each configured to discharge liquid eluted from the extracellular vesicle capturing sections 16 to 18 and including the microRNA, the extracellular vesicle capturing sections 16 to 18 being positioned between the introduction section 14 and the one or more discharge sections 20a to 20c; second flow paths 15a to 15c between the introduction section 14 and the extracellular vesicle capturing sections 16 to 18; and third flow paths 19a to 19c between the extracellular vesicle capturing sections 16 to 18 and the one or more discharge sections 20a to 20c, wherein the one or more discharge sections 20a to 20c correspond in number to the one or more immobilization supports.

With the above arrangement, in a case where a sample includes an extracellular vesicle, it is possible to collect a microRNA contained in the exosome and a microRNA contained in the microparticle separately each in correspondence with a surface sugar chain included in the extracellular vesicle. This makes it possible to accurately diagnose cancer with high specificity with use of a simple structure as described above.

The liquid delivery system 11 delivers a sample, from which a microRNA is to be detected, through a first flow path 13 to an introduction section 14 on the plate 12. The sample is preferably one or more kinds of samples selected from the group consisting of blood, blood plasma, blood serum, saliva, tear, swab, phlegm, urine, spinal fluid, amniotic fluid, synovial fluid, ascitic fluid, and pleural fluid.

The liquid delivery system 11 includes (i) a liquid reservoir section (not shown) capable of storing liquid and (ii) a pressure transmitting section (not shown) capable of transmitting external force (applied to the liquid delivery system 11) in the form of a pressure change in the liquid reservoir section. The pressure transmitting section causes liquid stored in the liquid reservoir section to flow into the first flow path 13 in response to a pressure change. The liquid is, for example, the sample, cleaning fluid, or a surfactant for use in extracting a microRNA.

The sample caused to flow into the first flow path 13 by the liquid delivery system 11 runs from the introduction section 14 into second flow paths 15a to 15c to reach respective extracellular vesicle capturing sections 16 to 18 placed in respective extracellular vesicle capturing section accommodating sections 16' to 18'. The extracellular vesicle capturing section 16 includes an immobilization support on which one or more kinds of high-mannose sugar chain binding lectins are immobilized. The extracellular vesicle capturing section 17 includes an immobilization support on which one or more kinds of sialyl Lewis sugar chain binding lectins are immobilized. The extracellular vesicle capturing section 18 includes an immobilization support on which one or more kinds of core α1-6 fucose binding lectins are immobilized.

The extracellular vesicle capturing sections 16 to 18, in other words, include respective immobilization supports corresponding to a high-mannose sugar chain, a sialyl Lewis sugar chain, and a core α1-6 fucose, each of which is a surface sugar chain included in an extracellular vesicle derived from a cancer cell.

A flow-through fraction that did not bind to the lectin flows through third flow paths 19a to 19c into respective discharge sections 20a to 20c, and goes on to flow from the discharge sections 20a to 20c through respective fourth flow paths 21a to 21c into respective waste liquid accommodating sections 23. In a case where a sample includes an extracellular vesicle that binds to the lectin, the extracellular vesicle, due to its surface sugar chain, binds to the lectin to be captured. Then, the immobilization supports included in the respective extracellular vesicle capturing sections are washed sufficiently. After that, surfactants having, for example, respective concentrations different from each other are passed through the respective immobilization supports as described under <1-2. Extracting microRNA from extracellular vesicle>. This allows (i) a microRNA extracted from a microparticle to be collected into accommodating sections 24 and (ii) a microRNA extracted from an exosome to be collected into accommodating sections 25.

The drawing shows a double-headed arrow near the microRNA accommodating section 22. This indicates that the microRNA accommodating section 22 is movable. For instance, after the finish of collection of waste liquid into the accommodating sections 23, the microRNA accommodating section 22 can be moved toward the plate 12 so that the respective open ends of the flow paths 21a to 21c are positioned substantially directly above the respective accommodating sections 24. Similarly, after the finish of collection of a microRNA extracted from a microparticle into the accommodating sections 24, the microRNA accommodating section 22 can be moved toward the plate 12 so that the respective open ends of the flow paths 21a to 21c are positioned substantially directly above the respective accommodating sections 25.

The microRNA collected in the accommodating sections 24 and the microRNA collected in the accommodating sections 25 are delivered to the detecting section described under <1-3. Detecting section for detecting microRNA>. The sample can be delivered to the detecting section through, for example, flow paths formed from the accommodating sections 24 and the accommodating sections 25 to the detecting section for liquid delivery. The sample may alternatively be collected from the accommodating sections 24 and the accommodating sections 25 into, for example, a sample tube temporarily for purification and then delivered to the detecting section.

The cancer diagnosis device 1, as described above, includes on a single plate 12 a plurality of immobilization supports on which different kinds of lectins are immobilized. This allows a sample to be switched within a reduced time period, and only requires a small amount of sample for examination about many items.

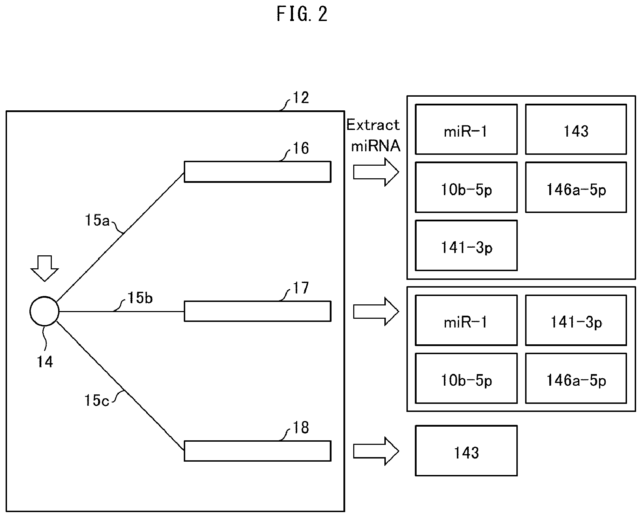

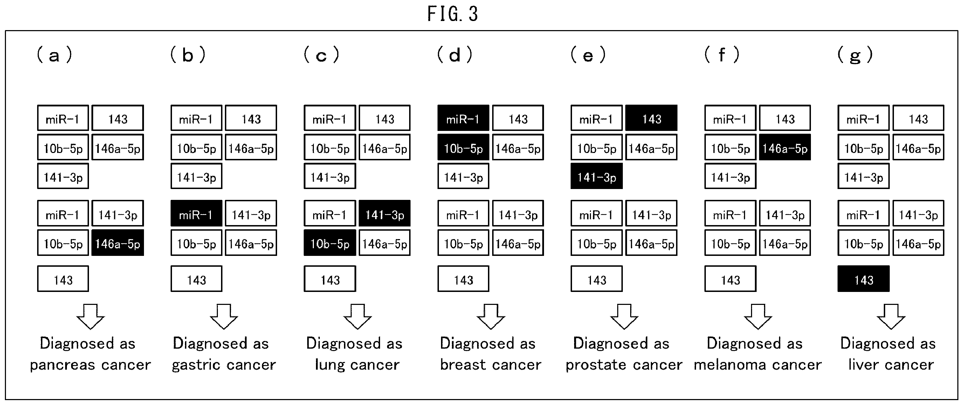

With reference to Figs. 2 and 3, the description below deals with how cancer diagnosis is carried out with use of a detecting section in the form of an array to which probes are fixed for detecting microRNAs included in the extracellular vesicles.

Fig. 2 is a diagram schematically illustrating an example of how extracted liquid extracted from the extracellular vesicle capturing sections 16 to 18 is fed to an array to which cDNA of microRNAs is fixed as probes. The members 14 to 18 in Fig. 2 represent the same members numbered as such in Fig. 1. The arrow above the introduction section 14 indicates that a sample is caused to flow into the introduction section 14. The squares in Figs. 2 and 3 each represent a region to which a probe is fixed and show the name of a microRNA used as a probe.

Fig. 2 indicates that (i) cDNA of miR-1 and 10b-5p, which are known as being included in an extracellular vesicle of a breast cancer cell, (ii) cDNA of 141-3p and 143, which are known as being included in an extracellular vesicle of a prostate cancer cell, and (iii) cDNA of 146a-5p, which is known as being included in an extracellular vesicle of a melanoma cell, are each fixed to the array as a probe and that extracted liquid extracted from the extracellular vesicle capturing section 16, which includes an immobilization support on which a high-mannose sugar chain binding lectin is immobilized, is fed to the array. The extracellular vesicles of a breast cancer cell, a prostate cancer cell, and a melanoma cell each include a high-mannose sugar chain as a surface sugar chain.

Fig. 2 also indicates that (i) cDNA of 146a-5p, which is known as being included in an extracellular vesicle of a pancreas cancer cell, (ii) cDNA of miR-1, which is known as being included in an extracellular vesicle of a gastric cancer cell, and (iii) cDNA of 10b-5p and 141-3p, which are known as being included in an extracellular vesicle of a lung cancer cell, are each fixed as a probe and that extracted liquid extracted from the extracellular vesicle capturing section 17, which includes an immobilization support on which a sialyl Lewis sugar chain binding lectin is immobilized, is fed to the array. The extracellular vesicles of a pancreas cancer cell, a gastric cancer cell, and a lung cancer cell each include a sialyl Lewis sugar chain as a surface sugar chain.

Fig. 2 also indicates that cDNA of 143, which is known as being included in an extracellular vesicle of a liver cancer cell, is fixed as a probe and that extracted liquid extracted from the extracellular vesicle capturing section 18, which includes an immobilization support on which a core α1-6 fucose binding lectin is immobilized, is fed to the array. The extracellular vesicle of a liver cancer cell includes a core α1-6 fucose as a surface sugar chain.

Fig. 3 is a diagram schematically illustrating an example of how cancer diagnosis is carried out on the basis of the result of observation of fluorescence from the array. In (a) to (g) of Fig. 3, each square with a gray background indicates that fluorescence has been observed. In (a) of Fig. 3, fluorescence has been observed in only 146a-5p of the panel to which the extracted liquid extracted from the extracellular vesicle capturing section 17 has been fed. This indicates that the extracted liquid includes 146a-5p as a microRNA. Since the microRNA extracted from an extracellular vesicle including a sialyl Lewis sugar chain as a surface sugar chain is 146a-5p, the sample donor can, in this case, be diagnosed as being affected with pancreas cancer.

In (d) of Fig. 3, fluorescence has been observed in miR-1 and 10b-5p of the panel to which the extracted liquid extracted from the extracellular vesicle capturing section 16 has been fed. This indicates that the extracted liquid includes miR-1 and 10b-5p each as a microRNA. Since the microRNAs each extracted from an extracellular vesicle including a high-mannose sugar chain as a surface sugar chain are miR-1 and 10b-5p, the sample donor can, in this case, be diagnosed as being affected with breast cancer.

Similarly, the result of (b) of Fig. 3 allows the sample donor to be diagnosed as being affected with gastric cancer. The result of (c) of Fig. 3 allows the sample donor to be diagnosed as being affected with lung cancer. The result of (e) of Fig. 3 allows the sample donor to be diagnosed as being affected with prostate cancer. The result of (f) of Fig. 3 allows the sample donor to be diagnosed as being affected with melanoma. The result of (g) of Fig. 3 allows the sample donor to be diagnosed as being affected with liver cancer.

Fig. 3 merely shows an example diagnosis. A cancer diagnosis device in accordance with an embodiment of the present invention is capable of specifically separating an extracellular vesicle of a cancer cell on the basis of the surface sugar chain included in the extracellular vesicle. This makes it possible to rapidly determine the kind of microRNA contained in the extracellular vesicle. The cancer diagnosis device is thereby capable of producing a particularly remarkable effect of discovering initial occurrence, recurrence, and metastasis of cancer organ-specifically at a stage where the cancer is small.

The present invention is not limited to the embodiments, but can be altered by a skilled person in the art within the scope of the claims. The present invention also encompasses, in its technical scope, any embodiment derived by combining technical means disclosed in differing embodiments.

### Examples

The description below deals with the present invention on the basis of Examples. The present invention should, however, not be construed as being limited to the Examples.

### Example 1: Capture of Exosomes from Human Melanoma Cell Culture Supernatant

Exosomes (and proteins expressed by the exosomes) were captured with use of an OAA immobilized spin column. The OAA immobilized spin column was obtained by immobilizing OAA, which is a type-I lectin, to a silica monolith, which is an immobilization support filled into a spin column. Tests were carried out three times, and results are shown as an average thereof.

Used as the OAA immobilized spin column was an AIST-OAA1 immobilized spin column (diameter: 4 mm; thickness: 2 mm; capacity: 27 µl; immobilized amount of OAA1: 42 µg; available from Kyoto Monotech Co., Ltd.). Used as comparative columns were: (i) an anti-CD9 antibody immobilized spin column (capacity: 27 µl; Exo Trap (registered trademark) Exosome Isolation Spin Column Kit for Protein Research; available from Cosmo Bio Co., Ltd.); and (ii) phosphatidylserine binding Tim4 magnetic beads (PS affinity beads) (MagCapture (registered trademark) Exosome Isolation Kit PS; available from WAKO).

For each spin column, (i) preservative solution in the spin column was centrifugally removed (4°C, 1500 × g, 30 seconds), (ii) centrifugal washing was performed thrice in the same manner with 200 µl of ultrapure water, and then (iii) 200 µl of equilibration buffer solution (20 mM PB (pH7.4), 500 mM NaCl) was added and centrifugal separation carried out (4°C, 1500 × g, 30 seconds). This process was carried out a further two more times so that equilibration was achieved.

### 1. Method of Preparing Sample

Used as melanoma cells was A375 (ATCC (registered trademark) CRL-1619) purchased from ATCC.

The cells were cultured for five days in a DMEM culture medium (Dulbecco's Modified Eagle Medium, available from Sigma) which contained (i) 10% (v/v) bovine-derived exosome-depleted FBS (Exosome-depleted FBS Media Supplement (System Bioscience)), (ii) 100 U/ml of penicillin and (iii) 100 µg/ml of streptomycin. Once the melanoma cells had reached a confluent state, 792 ml of the culture solution was subjected to stepwise fractionation via centrifugal separation. Specifically, after 10 minutes of centrifugal separation at 300 g, a first supernatant was collected. This first supernatant was further subjected to 10 minutes of centrifugal separation at 2,000 g, to obtain a second supernatant. This second supernatant was further subjected to 30 minutes of centrifugal separation at 10,000 g, to obtain a third supernatant. In this way, separation was achieved between a cell fraction and a supernatant (centrifuge supernatant).

The cell fraction was suspended in 10 ml of a phosphate buffer solution (phosphate-buffered saline, PBS) and was then thrice subjected to 10 minutes of centrifugal washing at 300 g.

After washing, the cell fraction was suspended in 10 ml of PBS. The number of cells in the suspension was counted with use of a cell counter. After the number of cells were counted, the suspension was subjected to 10 minutes of centrifugal separation at 300 g. A resultant cell residue was then dissolved in 1 ml of a RIPA buffer solution (50 mM Tris-HCl, 150 mM NaCl, 5 mM EDTA, 1% NP-40, 1 mM PMSF, 1 mM DTT, and 1 mg/ml of protease inhibitor).

From the centrifuge supernatant obtained as above, a 100 ml portion (centrifuge supernatant A) was used for preparation of ultracentrifugation fraction, and a 692 ml portion (centrifuge supernatant B) was used for extracellular vesicle capture. The centrifuge supernatant A was subjected to 70 minutes of ultracentrifugation at 100,000 g. After ultracentrifugation, 500 µl of PBS was added to a pellet containing extracellular vesicles. This was considered to be an extracellular vesicle fraction (ultracentrifugation fraction). From this extracellular vesicle fraction, an amount equivalent to 1 ml of the centrifuge supernatant was considered to be an ultracentrifugation fraction 1.

The centrifuge supernatant B was filtered through a filter (polyether sulfone, DISMIC 25SS045RS, available from ADVANTEC) having a pore diameter of 0.45 µm. Thereafter, a resultant filtrate was applied to three of the above-described AIST-OAA1 spin columns (respectively referred to as AIST-OAA spin columns 1 through 3), and to two anti-CD9 antibody immobilized spin columns (respectively referred to as anti-CD9 antibody immobilized spin columns 1 and 2), in an amount of 600 µl per spin column. Thereafter, centrifugal separation at 1500 g was carried out for 30 seconds. The application and the centrifugal separation were carried out ten times. Next, each column was subjected to a washing step in which 200 µl of PBS was added and then centrifugal separation at 1500 g was carried for 30 seconds. This washing step was carried out three times.

Thereafter, to each of the AIST-OAA spin column 1 and the anti-CD9 antibody immobilized spin column 1 was added 100 µl of an SDS-PAGE sample buffer solution (2% SDS, 6% glycerol, 0.005% bromophenol blue, and 50 mM Tris-HCl (pH 6.8)). To each of the AIST-OAA spin column 2 and the anti-CD9 antibody immobilized spin column 2 was added 100 µl of a quantitative PCR buffer solution (QIAzol lysis buffer, available from Qiagen). To the AIST-OAA spin column 3 was added 100 µl of the SDS-PAGE sample buffer solution. Each column was allowed to stand for 30 minutes. Thereafter, 30 seconds of spindown at 400 g was carried out, and an eluate was obtained. Because the tests were carried out three times, three sets of each of the AIST-OAA spin columns 1 through 3 and the anti-CD9 antibody immobilized spin columns 1 and 2 were prepared.

The eluates obtained from the AIST-OAA1 spin columns 1 through 3 are referred to as eluates 2a through 2c, respectively. The eluates obtained from the anti-CD9 antibody immobilized spin columns 1 and 2 are referred to as eluates 3a and 3b, respectively.

Furthermore, 6 ml of the filtrate was subjected to ultrafiltration with use of an ultrafiltration membrane (10K, PLGCO4310, available from Amicon). Then, 60 µl of a resultant ultrafiltration fraction was added 60 µl of the PS affinity bead suspension. The ultrafiltration fraction was then allowed to stand at room temperature for 3 hours. Thereafter, a washing process was carried out three times according to a manual (Exosome Isolation Kit PS). After the PS affinity beads were washed, a step involving (i) adding 50 µl of an exosome elution buffer (containing a chelating agent; buffer available from Cosmo Bio), and (ii) carrying out elution in accordance with the manual was performed twice, so that an eluate 4 was obtained.

### 2. SDS-PAGE

The ultracentrifugation fraction 1 and the eluates 2a, 3a, and 4 were subjected to SDS-PAGE. The SDS-PAGE was carried out in accordance with the method of Schagger and Jagow (1987), which is a method improved for measurement of low molecular weight proteins. The SDS-PAGE was carried out with use of a 12% acrylamide gel, in a tris-tricine based buffer solution (3.0 M of Tris-HCl, 0.3% SDS, pH 8.45). A positive electrode buffer solution (0.2 M of Tris-HCl, pH 8.9) was used as a lower electrode liquid. A negative electrode buffer solution (0.1 M of Tris-HCl, 0.1 M of tricine, 0.1% SDS, pH 8.25) was used as an upper electrode liquid. After a SDS-PAGE sample buffer solution was added to the sample, heat treatment was carried out for 5 minutes at 100°C to obtain a sample for electrophoresis. A gel was set in an electrophoresis device (AE-6530, ATTO), and an electric current was applied for 90 minutes at a constant voltage (CV) of 100 V. After the electrophoresis, protein staining was carried out on the gel with use of Coomassie brilliant blue. The respective added amounts of the ultracentrifugation fraction 1 and the eluates 2a, 3a, and 4 were each equivalent to 1 ml of the centrifuge supernatant of the culture solution.

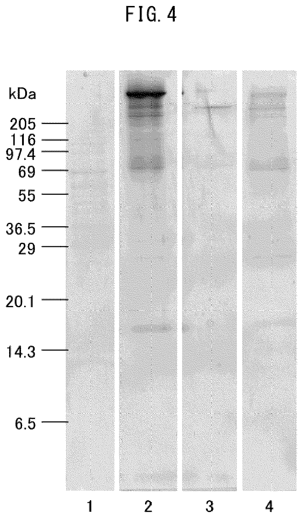

Fig. 4 indicates results of the SDS-PAGE. In Fig. 4, lanes 1 through 4 indicate the results for the ultracentrifugation fraction 1, and the eluates 2a, 3a, and 4, respectively.

From Fig. 4, it can be seen that the eluate 2a obtained from the AIST-OAA1 spin column contains many protein components. In contrast, it can be seen that in the eluate 3a obtained from the anti-CD9 antibody immobilized spin column and the eluate 4 obtained from the PS affinity beads, both the variety and amount of proteins contained was low.

### 3. Western Blot Using Anti-CD9 Antibody as Primary Antibody

Next, the ultracentrifugation fraction 1 and the eluates 2a, 3a, and 4 were subjected to Western blotting, with use of (i) mouse anti-CD9 antibody (available from BD Pharmagen) diluted by a factor of 5000, as a primary antibody and (ii) HRP (horseradish peroxidase)-labeled goat anti-mouse IgG antibody (available from Santa Cruz Biotechnology) diluted by a factor of 10,000, as a secondary antibody. The respective added amounts of the ultracentrifugation fraction 1 and the eluates 2a, 3a, and 4 were each equivalent to 1 ml of the centrifuge supernatant of the culture solution.

A wet transfer device "Mini Trans0BrotR Cell Module" (Bio-Rad) was used to transfer the SDS polyacrylamide gel that had been subjected to electrophoresis to a PVDF membrane (Takara Bio Inc.). After transfer, a 5% blocking buffer solution (5% skim milk, TBS-T (20 mM Tris-HCl (pH 8.0), 0.15 M NaCl, 0.05% Tween 20)) was added to the PVDF membrane. The PVDF membrane was then allowed to stand at room temperature for 1 hour. After blocking was finished, the PVDF membrane was reacted, for 16 hours at 4°C, with the primary antibody diluted in a 2.5% blocking buffer solution.

Next, after the PVDF membrane was washed five times with TBS-T, the secondary antibody, which was diluted with a 2.5% blocking buffer solution was added, and then incubation was carried out (room temperature, 90 minutes). The PVDF membrane was then washed five times with TBS-T, immersed in ECL (available from Bio Rad), and reacted for 5 minutes. Chemiluminescence was detected with use of a Las-3000 (available from FujiFilm Corporation).

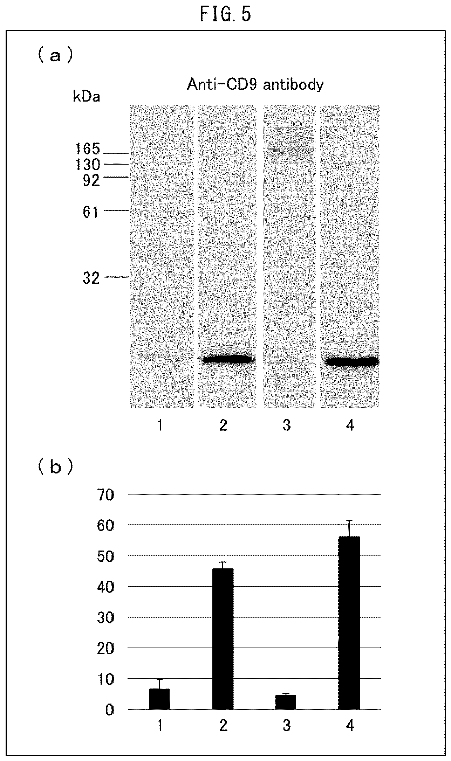

Fig. 5 indicates results of the Western blot. In Fig. 5, numbers 1 through 4 indicate the results for the ultracentrifugation fraction 1, and the eluates 2a, 3a, and 4, respectively. (a) of Fig. 5 indicates the results of immunostaining. (b) of Fig. 5 indicates the results of quantification of a component (i.e., CD9) which was positive for staining in the immunostaining. In (b) of Fig. 5, the vertical axis represents a relative ratio of luminescence amount of CD9 after immunostaining (i.e., brightness after addition of chemiluminescence agent).

The results of the quantification were obtained by using the image processing software ImageJ and performing measurements in accordance with the manual for ImageJ.

In (a) of Fig. 5, a band around 24 kDa indicates the results of CD9 detection. A small amount of CD9 was detected in the ultracentrifugation fraction 1. In comparison to the eluate 3 from the anti-CD9 antibody immobilized spin column, a large amount of CD9 was detected in the eluate 2a from the AIST-OAA1 spin column. In the eluate 3a, a high molecular weight band is detected around 165 kDa, but this band is derived from the anti-CD9 antibody (mouse IgG) contained in the anti-CD9 antibody immobilized spin column. The anti-CD9 antibody is an antibody against the antigen CD9 common in exosomes.

The captured amount of CD9 was highest in the eluate 4. However, as can be seen from the results indicated in Figs. 4 and 5, a greater amount of CD9 was captured with use of the AIST-OAA1 spin column as compared to the anti-CD9 antibody immobilized spin column. This demonstrates that (i) the AIST-OAA1 spin column enables a greater amount of exosome capture than the anti-CD9 antibody immobilized spin column, and (ii) the AIST-OAA1 spin column enables a greater amount of exosome capture as compared to a preparation involving ultracentrifugation, despite involving an exceedingly shorter amount of treatment time. These results indicate that OAA makes it possible to efficiently capture exosomes derived from cancer cells.

Note that PS affinity beads (coated with phosphatidylserine binding Tim4) have affinity to phosphatidylserine, which is a membrane component of extracellular vesicles (exosomes, microparticles). As such, PS affinity beads presumably capture a wide range of extracellular vesicles. In contrast, an AIST-OAA1 spin column captures only extracellular vesicles which have high-mannose sugar chains on their membranes. As such, it is surmised that the AIST-OAA1 spin column is capable of selectively capturing specific exosomes. Therefore, in a case where an AIST-OAA1 spin column is used, there will be a lower detected amount of CD9 as compared to a case where PS affinity beads are used, but the AIST-OAA1 spin column is presumably more suitable than PS affinity beads for capturing exosomes.

### 4. Western Blot Using Anti-CD63 Antibody

The ultracentrifugation fraction 1 and the eluates 2a, 3a, and 4 were subjected to Western blotting, with use of (i) mouse anti-CD63 antibody (available from Santa Cruz Biotechnology) as a primary antibody and (ii) HRP-labeled goat anti-mouse IgG antibody (available from Santa Cruz Biotechnology) as a secondary antibody. The respective added amounts of the ultracentrifugation fraction 1 and the eluates 2a, 3a, and 4 were each equivalent to 1 ml of the centrifuge supernatant of the culture solution. Similar to the anti-CD9 antibody, the anti-CD63 antibody is an antibody against the antigen CD63 common in exosomes. CD63 has a molecular weight of 34 kDa to 55 kDa.

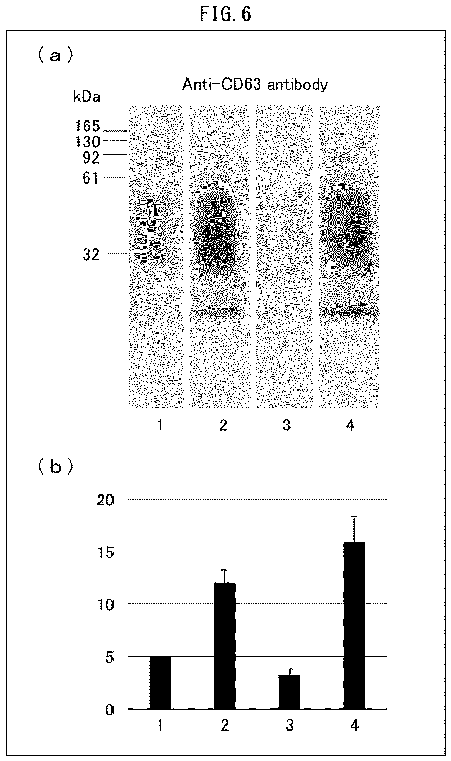

Fig. 6 indicates results of the Western blot. In Fig. 6, numbers 1 through 4 indicate the results for the ultracentrifugation fraction 1, and the eluates 2a, 3a, and 4, respectively. (a) of Fig. 6 indicates the results of immunostaining. (b) of Fig. 6 indicates the results of quantification of a component (i.e., CD63) which was positive for staining in the immunostaining. In (b) of Fig. 6, the vertical axis represents a relative ratio of luminescence amount of CD63 after immunostaining (i.e., brightness after addition of chemiluminescence agent).

The results of the quantification were obtained by using the image processing software ImageJ and performing measurements in accordance with the manual for ImageJ.

As indicated by (a) of Fig. 6, almost no CD63 was detected in the eluate 3a from the anti-CD9 antibody immobilized spin column, but a large amount of CD63 was detected in the eluate 2a from the AIST-OAA1 spin column.

The captured amount of CD63 was highest in the eluate 4. However, as can be seen from the results indicated in Figs. 4 and 6, a greater amount of CD63 was captured with use of the AIST-OAA1 spin column as compared to the anti-CD9 antibody immobilized spin column. This demonstrates that (i) the AIST-OAA1 spin column enables a greater amount of exosome capture than the anti-CD9 antibody immobilized spin column, and (ii) the AIST-OAA1 spin column enables a greater amount of exosome capture as compared to a preparation involving ultracentrifugation, despite involving an exceedingly shorter amount of treatment time. These results indicate that OAA makes it possible to efficiently capture exosomes derived from cancer cells.

Note that PS affinity beads have affinity to phosphatidylserine, which is a membrane component of extracellular vesicles (exosomes, microparticles). As such, PS affinity beads presumably capture a wide range of extracellular vesicles. In contrast, an AIST-OAA1 spin column captures only extracellular vesicles which have high-mannose sugar chains on their membranes. As such, it is surmised that the AIST-OAA1 spin column is capable of selectively capturing specific exosomes. Therefore, in a case where an AIST-OAA1 spin column is used, there will be a lower detected amount of CD63 as compared to a case where PS affinity beads are used, but the AIST-OAA1 spin column is presumably more suitable than PS affinity beads for capturing exosomes.

### Example 2: Quantification of MicroRNA Contained in Exosomes Derived from Human Melanoma Cells

RT-PCR was used to quantify microRNA contained in the exosomes obtained in Example 1. MicroRNA was extracted from the exosomes with use of an RNA binding spin column (available from Qiagen, miRNeasy Serum/Plasma Kit), and using RNase free water to elute the microRNA captured in the column. RT-PCR was performed after reaction with a high-capacity reverse transcriptase, the real time PCR being used to detect microRNA levels so as to quantify the microRNA.

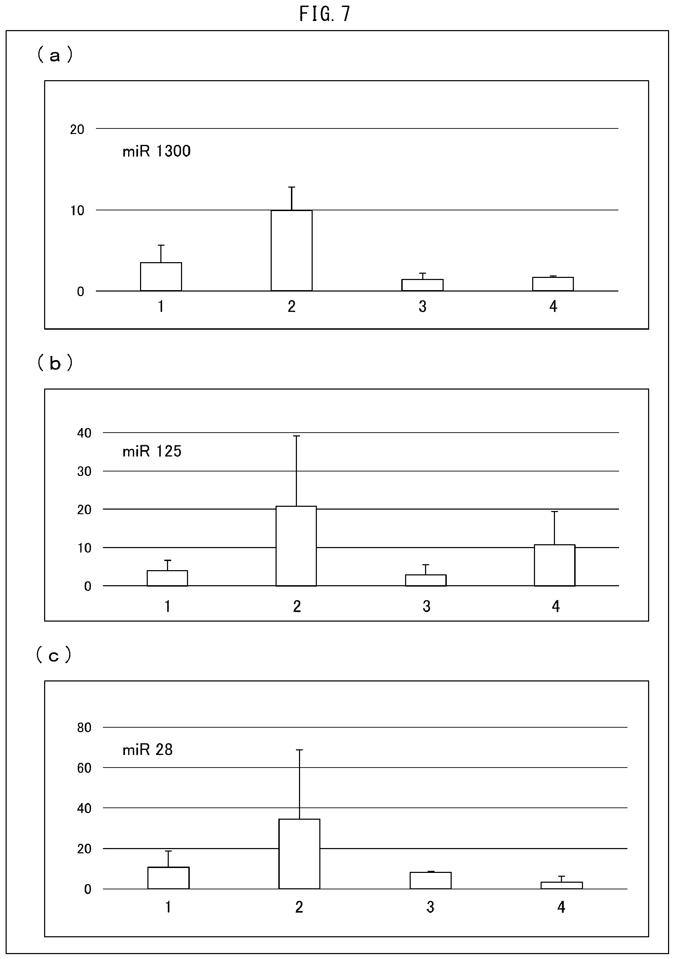

Fig. 7 indicates results of comparing amounts of microRNA contained in fractions in which the exosomes are collected. (a), (b), and (c) of Fig. 7 indicate comparisons of amounts of miR-1300, miR-125, and miR-28, respectively. In Fig. 7, numbers 1 to 4 indicate the ultracentrifugation fraction 1 and the eluates 2b, 3b, and 4, respectively. The vertical axis represents a relative ratio of the microRNA amount (in other words, a relative ratio of microRNA expression).

The microRNAs miR-1300, miR-125, and miR-28 are each expressed in a wide range of cancer cells. However, miR-1300 is expressed comparatively more in colon cancer cells, miR-125 is expressed comparatively more in breast cancer cells, and miR-28 is expressed comparatively more in renal cell cancer cells.

As illustrated in Fig. 7, miR-1300, miR-125, and miR-28 were each found to be contained in markedly high amounts in the eluate 2b obtained from the AIST-OAA1 spin column.

Detected amounts of CD9 and CD63 were highest in the eluate 4 obtained using PS affinity beads, as indicated in Figs. 5 and 6. However, the amounts of miR-1300, miR-125, and miR-28 contained in the eluate 4 were each lower than in the eluate 2b. As described above, it is surmised that the AIST-OAA1 spin column is capable of selectively capturing specific exosomes. As such, it is inferred that the results shown in Fig. 7 are based on the fact that the AIST-OAA1 spin column selectively captured exosomes.

From the results of Examples 1 and 2, it can be seen that (i) a method of purifying exosomes with use of an AIST-OAA1 spin column makes it possible to purify exosomes more efficiently than existing purification methods which use an anti-CD9 antibody immobilized spin column or ultracentrifugation, and (ii) the profile of microRNA of an exosome collected in an eluate differs depending on the purification method, and that an AIST-OAA1 spin column is capable of specifically capturing exosomes derived from cancer cells.

Furthermore, from the results of Examples 1 and 2, it is predicted that the profiles of microRNA of exosomes differ not only when purified with use of OAA, but also when purified with use of columns in which differing types of high-mannose sugar chain specific lectins (lectins whose respective recognition sites of branched sugar chains differ) are immobilized. In other words, using such columns enables fractionation based on differences in surface sugar chain structures of exosomes.

### Example 3: Purification of Exosomes by Use of AIST-OAA1 Spin Column

To the eluate 2c obtained in Example 1, a 10% TCA/acetone solution was added in an amount of 10 times by volume. After the addition, the eluate 2c was allowed to stand for 1 hour at -20°C, and was then subjected to centrifugal separation (4°C, 15,000 × g, 10 minutes). After a supernatant was removed, 1 ml of ice-cold acetone was added to a precipitate. After the addition, the precipitate was allowed to stand for 10 minutes at -20°C. Thereafter, centrifugal separation was performed, and a supernatant removed. This washing process was repeated a further 9 times. Thereafter, the precipitate was air dried to obtain a dry sample, which was stored at -20°C.

To the dry sample, 50 mM ammonium bicarbonate (pH 8.0) was added so as to prepare a protein solution in an amount of 100 µg / 100 µl. To this protein solution (100 µl), 4.2 µl of 500 mM dithiothreitol was added. The protein solution was then heated for 1 hour at 60°C. Next, the solution was allowed to stand for 5 minutes at room temperature. Thereafter, 9 µl of 500 mM iodoacetamide was added. The solution was then allowed to stand in a dark place for 30 minutes at room temperature. Next, 2.3 µl of 50 mM ammonium bicarbonate (pH 8.0) was added so as to stop the alkylation reaction. Thereafter, 10% TCA/acetone was added in an amount of 10 times by volume. A precipitate that was produced was washed with acetone and then air dried. In this manner, the dry sample was reduced and alkylated so as to obtain an alkylate.

Next, a trypsin treatment was carried out. In other words, the alkylate was dissolved into 50 mM ammonium bicarbonate (pH 8.0), 0.5 µl of a trypsin solution (500 ng/µl, Sigma) was then added, and then incubation (37°C, overnight) was carried out. The solution was allowed to stand at -20°C so that the reaction stopped.

Next, a desalting treatment was carried out with use of a Zip Tip C18 (Millipore). First, a C18 support was activated (100% acetonitrile, 10 µl, three times) and equilibrated (0.1% formic acid, 10 µl, three times). To the sample that had been subjected to the trypsin treatment, 10 µl of formic acid was added, and sample peptides were adsorbed to the Zip Tip C18. Next, the sample peptides adsorbed to the Zip Tip C18 were eluted with 10 µl of 50% acetonitrile and 0.1% formic acid. A resultant eluate was air dried to obtain a dried product. The dried product was dissolved again in 0.1% formic acid so as to obtain a sample to be subjected to Nano LC-MS/MS analysis. The sample thus prepared was stored at 4°C until measurements were carried out.

The sample was subjected to Nano LC-MS/MS analysis with use of an Ultimate 3000RSLCnano (Thermo fisher Scientific) and an LTQ Orbitrap XL (Thermo Fisher Scientific). The Nano LC-MS/MS analysis was carried out by the Hiroshima University Natural Science Center for Basic Research and Development, Cryogenics and Instrumental Analysis Division, Materials Science Instrumental Analysis Section

For proteins (114 types) searched using Mascot search on the basis of a result of Nano LC-MS/MS analysis, databases such as Exocarta: Home-Exosome database (http://exocarta.org/) and Vesiclepedia: Home-Extracellular vesicles database (http://www.microvesicles.org/) were used to further determine whether or not these proteins were exosome derived. As a result, it was found that out of the 114 types of detected proteins, 110 types were known to be contained in an exosome.

Based on these results, it is presumed that use of an AIST-OAA1 spin column makes it possible to purify human melanoma cell-derived exosomes in a manner specific to high-mannose sugar chains, easily and in a very short amount of time.

### Example 4: Capture of Exosomes from Culture Supernatant of Various Types of Human Cancer Cells

The AIST-OAA1 immobilized spin column used in Example 1 was used to capture exosomes (and proteins expressed by the exosomes) from culture supernatants of melanoma cells, colon cancer cells, liver cancer cells, pancreas cancer cells, breast cancer cells, and prostate cancer cells. Tests were carried out three times, and results are shown as an average thereof. Used as a blank column of the AIST-OAA1 immobilized spin column was a polylysine coating column (available from Kyoto Monotech Co., Ltd.), which is the same as the AIST-OAA1 immobilized spin column except that AIST-OAA1 has not been immobilized thereto. The blank column was used after carrying out the equilibration described for Example 1.

### 1. Method of Preparing Sample

### (1) Human Cancer-Derived Cell Strains

Used as melanoma cells was the A375 used in Example 1. Used as colon cancer cells was HT-29 (ATCC (registered trademark) HTB-38) purchased from ATCC. Liver cancer cells (HepG2: JCRB1054), spleen cancer cells (MIAPaCa-2: JCRB0070), breast cancer cells (MCF-7: JCRB0134), and prostate cancer cells (PC-3: JCRB9110) were purchased from the JCRB Cell Bank.

### (2) Culture Medium

The A375, HT-29, and HepG2 cells were cultured with use of a DMEM culture medium (Dulbecco's Modified Eagle Medium (Sigma)) containing (i) 10% (v/v) fetal bovine blood serum (FBS, Biowest) and (ii) 1% (v/v) penicillin-streptomycin (Gibco).

The MIA PaCa-2 cells were cultured with use of an MEM culture medium (Minimum Essential Medium (Sigma)) containing (i) 10% (v/v) FBS, (ii) 1% (v/v) penicillin-streptomycin, and (iii) 2 mM L-glutamine (Wako).

The MCF-7 cells were cultured with use of an MEM-NEAA culture medium (Minimum Essential Medium) containing (i) 10% (v/v) FBS, (ii) 1% (v/v) penicillin-streptomycin, (iii) 2 mM L-glutamine, and (iv) 1% (v/v) MEM non-essential amino acids (Wako).

The PC-3 cells were cultured with use of a Ham's F12 culture medium (Ham's F12 Medium (Gibco)) containing (i) 7% (v/v) FBS and (ii) 1% (v/v) penicillin-streptomycin.

Note that in final subculture solutions used for collection of extracellular vesicles (EVs), EXO-FBS Exosome-depleted FBS (SBI) was used instead of FBS.

### (3) Culturing of Human Cancer-Derived Cell Strains

Into respective ones of 15 ml Falcon tubes was added 10 ml of an FBS-added culture medium (i.e., the respective culture mediums described in section (2) above) which had been warmed in a 37°C water bath. In parallel with this operation, storage tubs respectively containing frozen specimens of the human cancer-derived cell strains described in section (1) above were immersed in a 37°C water bath. Once the frozen cells were partially thawed, the cells were respectively added into the 10 ml FBS-added culture medium prepared earlier.

Each of the Falcon tubes having the cells added thereto was centrifuged (200 × g, 4°C, 5 minutes). Thereafter, a supernatant was removed with an aspirator. Further, to each pellet (cells), 1 ml of the FBS-added culture medium was added, and the cells were suspended so as to prepare a cell suspension. This cell suspension was inoculated into a petri dish (IWAKI) measuring 60 mm in diameter.

To each of the petri dishes, 5 ml of the FBS-added culture medium was added. Each petri dish was slowly shaken so as to disperse the culture cells evenly in the petri dish. Next, each of the petri dishes was allowed to stand in an incubator (5% CO2, HERAcell) at 37°C and was cultured for approximately 3 to 4 days until a confluent state (80% to 90%) was achieved. During this time, once every two days, the culture medium was replaced and 6 ml of FBS-added culture medium was newly added.

After it was confirmed that the cells had reached a confluent state, the old culture solution in each of the petri dishes was suctioned and removed with use of an aspirator. Thereafter, the cells were washed with 10 ml of a phosphate buffer solution (pH 7.4). The washing fluid was suctioned and removed with use of an aspirator. Thereafter, 1 ml of a 1/10 dilution of 0.5% trypsin EDTA (5.0 g/L of trypsin, 2.0 g/L of EDTA·4Na, 8.5 g/L of NaCl; Gibco) was added and allowed to reach all cells. Thereafter, the dilution was immediately suctioned and removed from each petri dish (trypsin treatment).

Each of the petri dishes was then returned to the incubator at 37°C and allowed to stand for 5 minutes. After it was confirmed that the cells had begun detaching, the side of each flask was gently tapped to completely detach the remaining cells. Next, into each petri dish containing the trypsin treated cells was added 1 ml of the FBS-added culture medium. After the cells were gently suspended, each type of cell was inoculated into a respective petri dish (IWAKI) which measured 100 mm in diameter and contained 9 ml of the FBS-added culture medium.

Each of the petri dishes was allowed to stand in an incubator at 37°C for approximately 3 to 4 days until the cells reached a confluent state. During this time, the 10 ml of the FBS-added culture medium was replaced once every two days. By this method, the culture cells in a confluent state were subcultured. For each cell species, upon each subculture the number of petri dishes (measuring 100 mm in diameter) was doubled. This subculturing was continued, and for each cell species, after approximately 12 to 16 days when the number of culture petri dishes had reached 16, the subculturing proceeded to a final subculture. Note that in the subculturing, the phosphate buffer solution (WAKO) and the FBS-added culture medium to be used were warmed in advance in a 37°C water bath for 30 minutes.

Before the final subculture, the phosphate buffer solution and the EXO-FBS Exosome-depleted FBS culture mediums to be used were warmed in a 37°C water bath for 30 minutes. The EXO-FBS Exosome-depleted FBS culture mediums used were similar to the respective culture mediums described in section (2) above, except that the FBS was replaced with EXO-FBS Exosome-depleted FBS, which is FBS in which exosomes are depleted. The amount of the EXO-FBS Exosome-depleted FBS contained in each of culture medium was the same as the amount described in section (2) above. For example, in the DMEM culture medium described in (2), which was used as the culture medium for A375, 10% (v/v) EXO-FBS Exosome-depleted FBS was used instead of 10% (v/v) FBS.

After it was confirmed that the cells had reached a confluent state, 0.5% trypsin EDTA was used to detach the cells in the petri dish in accordance with the method for subculturing. To the trypsin treated culture cells, 10 ml of phosphate buffer solution was added, and the cells were gently suspended. Thereafter, the cell suspension was collected in a 50 ml Falcon tube. To the cell suspension, 40 ml of phosphate buffer solution was added, and centrifugation was carried out. Thereafter, an aspirator was used to suction and remove a supernatant. This washing process was carried out a total of 3 times, so that the trypsin and the FBS were removed.

After washing, 25 ml of a phosphate buffer solution was added to each pellet (cells), and the cells were gently suspended. Thereafter, a cell counter was used to count the number of cells. Each cell suspension was adjusted so that the total number of cells therein was 2.5 × 10⁷. After adjustment of cell number, each cell suspension was centrifuged. Thereafter, a supernatant was suctioned and removed by use of an aspirator. Thereafter, 25 ml of EXO-FBS Exosome-depleted FBS culture medium was added to each pellet, and the cells were suspended (2.5 × 10⁷ cells / 25 ml).

Next, petri dishes each measuring 100 mm in diameter and containing 9 ml of the culture medium were prepared, and the cell suspensions were inoculated into respective petri dishes such that there were 1.0 × 10⁶ cells per petri dish. Each petri dish was then allowed to stand in an incubator at 37°C for 48 hours. Supernatants of each of these culture solutions was collected via electric pipetter into a 50 ml Falcon tube and then stored at 4°C. Next, the EXO-FBS Exosome-depleted FBS culture medium was added in an amount of 10 ml per culture petri dish, and each petri dish was cultured in an incubator at 37°C for 48 hours. A supernatant of each culture solution was collected via electric pipetter and added to the culture supernatant obtained earlier.

The culture supernatant thus obtained was subjected to stepwise centrifugation (300 × g for 10 minutes, 2000 × g for 20 minutes, and 10,000 × g for 30 minutes), and a supernatant was then collected and filtered through a 0.20 µm filter (available from Advantec). The resultant substance was stored at -80°C as a culture supernatant. The culture supernatant obtained in this way was considered to be a clarified cancer cell culture supernatant.

The clarified cancer cell culture supernatants were collected in the following amounts. A375: 750 ml, MCF-7: 360 ml, HT-29: 330 ml, PC-3: 330 ml, HepG2: 420 ml, and MIA-PaCa2: 330 ml (Table 1).

**[Table 1]**

| Human cancer cell strain | Collected amount of clarified cancer cell culture supernatant (ml) | Ultracentrifugation fraction (EVs fraction) | | | |
|---|---|---|---|---|---|
| | | Used amount of clarified cancer cell culture supernatant (ml) | Collected amount of EVs fraction (µl) | Protein concentration (µg/µl) | Protein amount (µg) |
| Melanoma (A375) | 750 | 200 | 750 | 0.11 | 82.5 |
| Breast cancer (MCF-7) | 360 | 100 | 660 | 0.09 | 59.4 |
| Colon cancer (HT-29) | 330 | 70 | 650 | 0.05 | 32.5 |
| Prostate cancer (PC-3) | 330 | 100 | 500 | 0.05 | 25.0 |
| Liver cancer (HepG2) | 420 | 100 | 500 | 0.02 | 10.0 |
| Pancreas cancer (MIAPaCa-2) | 330 | 100 | 700 | 0.06 | 42.0 |

### (4) Preparation of Ultracentrifugation Fraction (Extracellular Vesicle Fraction) from Clarified Cancer Cell Culture Supernatant

The clarified cancer cell culture supernatants were set in an ultracentrifugal separation device (Himac) in the amounts indicated as "used amount (ml)" in Table 1. Ultracentrifugation (4°C, 100,000 × g, 70 minutes) was then carried out, and resultant supernatants discarded. To residue containing extracellular vesicles was added 10 ml of phosphate buffer. The residue was then pipetted, and then subsequently subjected to washing by further carrying out ultracentrifugation treatment in the same manner. After supernatant removal, the residue was suspended in a phosphate buffer and collected. This suspension was considered to be an ultracentrifugation fraction. Hereinafter, the ultracentrifugation fraction may also be referred to as a "extracellular vesicle fraction" or an "EVs fraction".

### 2. Quantification of Proteins in Ultracentrifugation Fraction

Amounts of proteins contained in the ultracentrifugation fractions derived from six types of cancer cells (ultracentrifugation fractions obtained in section 1. (4) above) were measured with use of a Micro BCA protein assay kit (Thermo Fischer Scientific). For the clarified cancer cell culture supernatants collected in section 1. (3) above, the amounts of proteins contained in the ultracentrifugation fraction obtained from the "used amount (ml)" indicated in Table 1 were as follows. A375: 82.5 µg, MCF-7: 59.4 µg, HT-29: 32.5 µg, PC-3: 25.0 µg, HepG2: 10.0 µg, and MIA-PaCa2: 42.0 µg.

### 3. Capture of Extracellular Vesicles (EVs) with Use of AIST-OAA1 Column

First, for HT-29 and MIA-PaCa2, respective clarified cancer cell culture supernatants (section 1. (3) above) and ultracentrifugation fractions prepared from the clarified cancer cell culture supernatants (section 1. (4) above) were prepared and added to respective AIST-OAA1 columns, so as to capture extracellular vesicles. The procedure of this is indicated in Fig. 9.

With reference to the protein amounts in the ultracentrifugation fractions as indicated in Table 1, (i) for each of the ultracentrifugation fractions, an amount of ultracentrifugation fraction containing 4 µg of protein was added to each of two AIST-OAA1 columns and two blank columns, and (ii) for each of the clarified cancer cell culture supernatants, an amount of the supernatant equivalent to 4 µg of protein of the ultracentrifugation fraction (8.6 ml in the case of HT-29; 9.56 ml in the case of MIA-PaCa2) was added to each of two AIST-OAA1 columns and two blank columns.

For the clarified cancer cell culture supernatants, the above amount was added to and passed through the columns in 600 µl portions at a time (4°C, 1500 × g, 30 seconds). After the sample was added, a washing process (4°C, 1500 × g, 30 seconds) using 200 µl of an equilibration buffer solution was carried out three times. Thereafter, extracellular vesicles which were adsorbed were solubilized using 100 µl of the below described solvent and eluted.

For Western blotting, an SDS electrophoresis buffer solution (4% SDS, 12% glycerol (w/v), 0.01% bromophenol blue, 50mM Tris-HCl (pH 6.8)) was added as a solubilizing agent to one AIST-OAA1 column and one blank column. For miRNA analysis, a QIAsol lysis buffer (Qiagen) was added as a solubilizing agent to one AIST-OAA1 column and one blank column. Each column was incubated for 30 minutes at room temperature. Thereafter, centrifugation (4°C, 400 × g, 30 seconds) was carried out, and a solubilized liquid was collected.

Next, with regard to the clarified cancer cell culture supernatants derived from A375, MCF-7, and PC-3 (section 1. (3) above), with reference to the protein amounts in the ultracentrifugation fractions as indicated in Table 1, an amount of each supernatant equivalent to 4 µg of protein of the ultracentrifugation fraction (9.68 ml in the case of A375; 6.72 ml in the case of MCF-7; 16 ml in the case of PC-3) was added to each of two AIST-OAA1 columns and two blank columns. Thereafter, adsorbed extracellular vesicles were solubilized and eluted using the same method as for HT-29 and MIA-PaCa2.

### 4. SDS-PAGE and Western Blotting

SDS-PAGE was carried out using the same conditions as for Example 1. Western blotting was carried using the same conditions as for Example 1, except that a mouse anti-CD81 antibody (available from Thermo Fisher Scientific) diluted by a factor of 5,000 was used as a primary antibody in addition to the mouse anti-CD9 antibody (available from BD Pharmagen) diluted by a factor of 5,000. The anti-CD9 antibody and the anti-CD81 antibody are antibodies against the antigens CD9 and CD81, respectively, which antigens are common in exosomes.

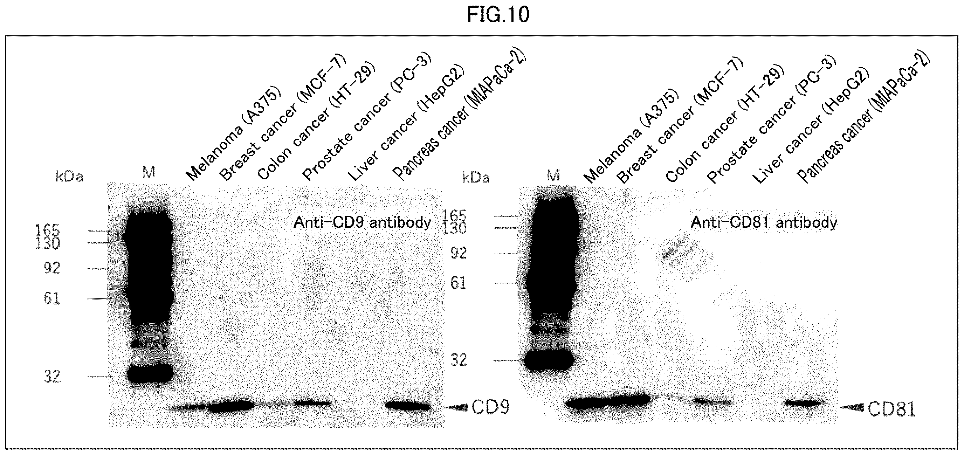

Western blots using anti-CD9 antibody and anti-CD81 antibody was carried out for the ultracentrifugation fractions derived from six types of cancer cells (ultracentrifugation fractions obtained in section 1. (4) above). Fig. 10 indicates results of the Western blots. As indicated in Fig. 10, band components (around a molecular weight of 24 kDa) which were positive for CD9 and CD81 were detected in all ultracentrifugation fractions except the ultracentrifugation fraction derived from HepG2. As such, it was clear that extracellular vesicles (exosomes) existed in five types of ultracentrifugation fractions, excluding the ultracentrifugation fraction derived from HepG2.

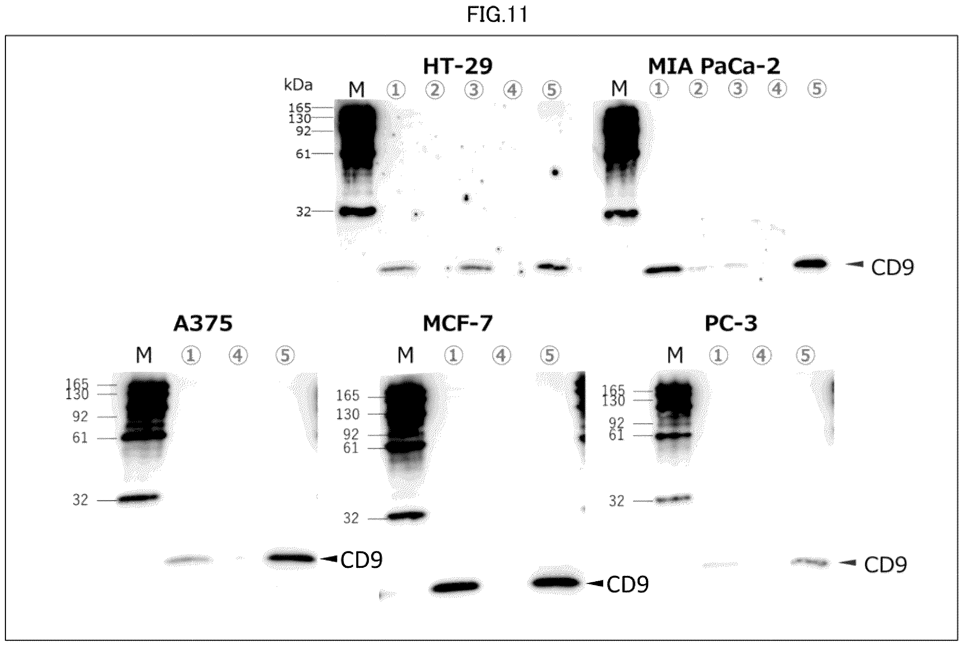

The eluates obtained from the AIST-OAA1 columns in section 3. above were subjected to Western blots using anti-CD9 antibody, and immunostaining was carried out. The results are indicated in Fig. 11. "M" denotes a marker, and the circled numbers represent lane numbers. These lane numbers correspond to the circled numbers in Fig. 9.

As indicated in Fig. 11, regarding HT-29 and MIA-PaCa2 cells, a positive band (approximately 24 kDa) was confirmed for (i) the ultracentrifugation fractions (lane 1, EVs fraction -1 in Fig. 9), (ii) the AIST-OAA1 column eluted fractions obtained after adding ultracentrifugation fractions to AIST-OAA1 columns (lane 3, EVs fraction -2 in Fig. 9), and (iii) the AIST-OAA1 column eluted fractions obtained after adding the clarified cancer cell culture supernatants to AIST-OAA1 columns (lane 5, EVs fraction -3 in Fig. 9). The positive band is denoted as "CD9" in Fig. 11.

As indicated in Fig. 11, regarding A375, MCF-7, and PC-3, a positive band (approximately 24 kDa) was confirmed for (i) the ultracentrifugation fractions (lane 1, EVs fraction -1 in Fig. 9), and (ii) the AIST-OAA1 column eluted fractions obtained after adding the clarified cancer cell culture supernatants to AIST-OAA1 columns (lane 5, EVs fraction -3 in Fig. 9).

### 5. Relative Quantitative Analysis of Exosomes

Relative quantitative analysis of EVs that were captured and collected was carried out based on the staining intensity of the anti-CD9 antibody positive band indicated in Fig. 11. This band indicates the existence of CD9, which is an antigen common to exosomes, and therefore presumably indicates the existence of exosomes.

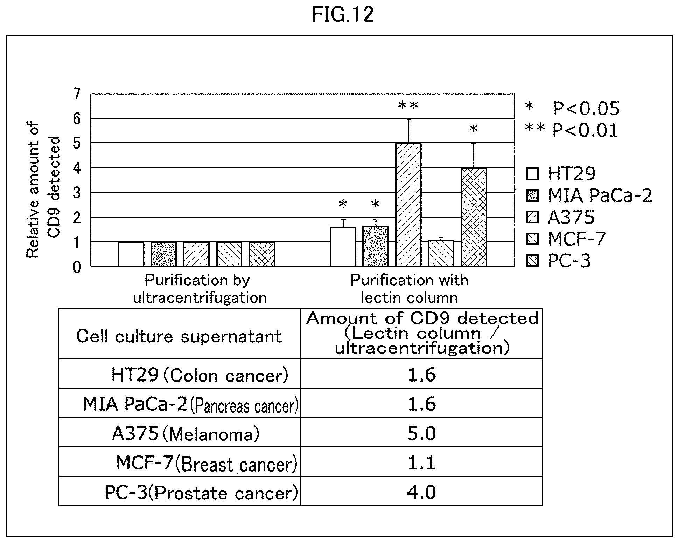

Fig. 12 indicates the results. The analysis was carried out with use of the image processing software ImageJ, in accordance with the manual for ImageJ. Fig. 12 indicates the results of quantification of staining-positive components (i.e., CD9) in the immunostaining of Fig. 11. The vertical axis represents a relative ratio of luminescence amount of CD9 after immunostaining (i.e., brightness after addition of chemiluminescence agent). This relative ratio is the luminescence amount (corresponding to lane 5 of Fig. 11) of CD9 in the eluate obtained via the method using the AIST-OAA1 column, where the luminescence amount (corresponding to lane 1 of Fig. 11) of CD9 in the ultracentrifugation fraction derived from cancer cells obtained by an ultracentrifugation method is considered to be 1. In Fig 12, "Purification by lectin column" indicates that purification was carried out with use of an AIST-OAA1 column.

As indicated in Fig. 12, it was found that the method using the AIST-OAA1 column makes it possible to collect exosomes in an amount that is 1.1 times to 5.0 times higher than in an ultracentrifugation method (which is a conventional standard method). Furthermore, while using the ultracentrifugation method took 4 hours, it was confirmed that a method using an AIST-OAA1 column makes it possible to prepare exosomes in an exceedingly short time of only 30 minutes.

### 6. Array Analysis of miRNA in Extracellular Vesicle

For A375, profile analysis was carried out regarding miRNA contained in (i) the ultracentrifugation fraction obtained by the ultracentrifugation method (lane 1 in Fig. 11) and (ii) the AIST-OAA1 column eluted fraction obtained after adding the clarified cancer cell culture supernatant to the AIST-OAA1 column (lane 5 in Fig. 11). The equipment used was the SurePrint G3 Human miRNA Microarray from Agilent. This array analysis corresponds to detection of 2,588 types of miRNA. With this array analysis, miRNA contained in samples can be detected semi-comprehensively and quantified.

Table 2 indicates the results of comparing (i) expression levels of miRNA in AIST-OAA1 column eluted fractions to (ii) expression levels of miRNA in the ultracentrifugation fractions, where the expression level of miRNA in the ultracentrifugation fraction is considered to be 1.

**[Table 2]**

| Expression level in AIST-OAA1 column eluted fraction compared to expression level in ultracentrifugation fraction | Number of sequences |
|---|---|
| Not less than 2 times | 23 |
| Not less than 10 times | 8 out of the above 23 |
| miRNA | Ratio of expression level in AIST-OAA1 column eluted fraction to expression level in ultracentrifugation fraction |
| hsa-miR-133b | 42.2 |
| hsa-miR-4732-5p | 28.2 |
| hsa-miR-130b-3p | 28.1 |
| hsa-miR-3185 | 26.9 |
| hsa-miR-3976 | 19.6 |

Table 2 indicates that there were 23 miRNA sequences for which the expression level in the AIST-OAA1 column eluted fraction was not less than 2 times the expression level in the ultracentrifugation fraction. There were 20 detected miRNA sequences for which the expression level in the AIST-OAA1 column eluted fraction was not less than 1 times the expression level in the ultracentrifugation fraction. Table 3 indicates (i) miRNAs for which the expression level in the AIST-OAA1 column eluted fraction was not less than 2 times the expression level in the ultracentrifugation fraction, and (ii) miRNAs for which the expression level in the AIST-OAA1 column eluted fraction was not less than 1 times the expression level in the ultracentrifugation fraction.

**[Table 3]**

| miRNA for which expression level in AIST-OAA1 column eluted fraction is not less than 2 times the expression level in ultracentrifugation fraction | miRNA for which expression level in AIST-OAA1 column eluted fraction is not less than 1 times the expression level in ultracentrifugation fraction |
|---|---|
| hsa-miR-133b | hsa-miR-4306 |
| hsa-miR-4732-5p | hsa-miR-575 |
| hsa-miR-130b-3p | hsa-miR-222-3p |
| hsa-miR-3185 | hsa-miR-320b |
| hsa-miR-3976 | hsa-miR-320d |
| hsa-miR-378d | hsa-miR-92a-3p |
| hsa-miR-6753-5p | hsa-miR-151b |
| hsa-miR-122-5p | hsa-miR-4534 |
| hsa-miR-1290 | hsa-miR-320a |
| hsa-miR-4793-5p | hsa-miR-6076 |
| hsa-miR-1246 | hsa-miR-320c |
| hsa-miR-675-3p | hsa-miR-22-3p |
| hsa-miR-210-3p | hsa-miR-30d-5p |
| hsa-miR-193a-3p | hsa-miR-130a-3p |
| hsa-miR-378i | hsa-miR-4741 |
| hsa-miR-376c-3p | hsa-miR-4687-5p |
| hsa-miR-4749-5p | hsa-miR-5189-3p |
| hsa-miR-378a-3p | hsa-miR-1229-3p |
| hsa-miR-371a-5p | hsa-miR-6798-3p |
| hsa-miR-4327 | hsa-miR-6889-3p |
| hsa-miR-1268a | |
| hsa-miR-423-5p | |
| hsa-miR-320e | |

As indicated in Table 2, there were 23 detected miRNA sequences for which the expression level in the AIST-OAA1 column eluted fraction was not less than 2 times the expression level in the ultracentrifugation fraction, and 8 detected miRNA sequences for which the expression level in the AIST-OAA1 column eluted fraction was not less than 10 times the expression level in the ultracentrifugation fraction. Furthermore, as indicated in Table 3, there were 20 detected miRNA sequences for which the expression level in the AIST-OAA1 column eluted fraction was not less than 1 times the expression level in the ultracentrifugation fraction. Among these miRNAs, the expression level of hsa-miR-133b was 42 times higher than in the ultracentrifugation fraction. Other miRNAs having high relative expression levels are indicated in Table 2. These results suggest that using an AIST-OAA column can potentially make it possible to selectively capture miRNA closely related to melanoma.

### Example 5: Analysis of Surface Sugar Chains of Extracellular Vesicles Captured by Column of Magnetic Beads Onto Which OAA Was Immobilized

### (1) Preparation of Extracellular Vesicles from A375

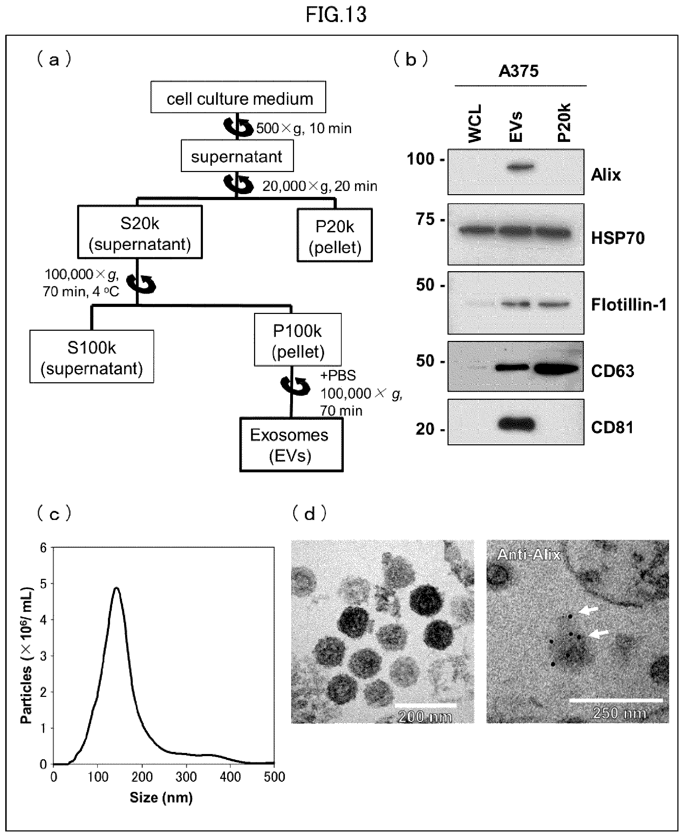

In order to analyze surface sugar chains of extracellular vesicles derived from melanoma, first an ultracentrifugation fraction was prepared from A375. Fig. 13 illustrates properties of extracellular vesicles of A375. (a) of Fig. 13 illustrates the procedure for preparing the ultracentrifugation fraction.

A375 was cultured in the same manner as in section 1. (3) of Example 4. A culture solution of a final subculture ("cell culture medium" in (a) of Fig. 13) was then centrifuged (500 × g, 10 minutes). A resultant supernatant was centrifuged (20,000 × g, 20 minutes), and a resultant supernatant ("S20k" in (a) of Fig. 13) was collected. This supernatant was set in an ultracentrifugal separation device (Himac), and ultracentrifugation (4°C, 100,000 × g, 70 minutes) was carried out. The resultant supernatant was discarded. To residue ("P100k" in (a) of Fig. 13) containing extracellular vesicles was added 10 ml of phosphate buffer. The residue was then pipetted, and then subsequently subjected to washing by further carrying out ultracentrifugation treatment in the same manner. After supernatant removal, the residue was suspended in a phosphate buffer and collected. This suspension was considered to be an ultracentrifugation fraction ("Exosomes (EVs)" in (a) of Fig. 13).

### (2) Western Blot of Extracellular Vesicles Derived from A375

A Western blot was carried out using the same conditions as for Example 1, except that the primary antibodies used were (i) mouse anti-Alix antibody (available from Santa Cruz Biotechnology), (ii) mouse anti-Hsp70 antibody (available from Santa Cruz Biotechnology), (iii) rabbit anti-Flotillin-1 antibody (available from Santa Cruz Biotechnology), (iv) mouse anti-CD63 antibody (available from Santa Cruz Biotechnology), and (iv) mouse anti-CD81 antibody (available from Santa Cruz Biotechnology). Alix, Hsp70, Flotillin-1, CD63, and CD81 are each antigens common in exosomes.

(b) of Fig. 13 indicates results of the Western blot. In (b) of Fig. 13, "WCL" denotes whole cell lysate. "EVs" denotes the ultracentrifugation fraction obtained in section (1) above. "P20k" corresponds to the P20k shown in (a) of Fig. 13. In the ultracentrifugation fraction obtained from A375, band components which were positive for Alix, Hsp70, Flotillin-1, CD63, and CD81 were detected. In other words, it was found that extracellular vesicles (exosomes) existed in the ultracentrifugation fraction.

### (3) Nanoparticle Tracking Analysis

The ultracentrifugation fraction (extracellular vesicle fraction) obtained in section (1) above was diluted in PBS such that the concentration of extracellular vesicles was the same as that in the culture solution of the final subculture.

The ultracentrifugation fraction diluted in this manner was subjected to nanoparticle tracking analysis with use of a nanoparticle analysis system Nano Sight (available from Malvern). A particle size distribution of extracellular vesicles derived from A375 was measured. (c) of Fig. 13 indicates the results of the nanoparticle tracking analysis. As indicated in (c) of Fig. 13, the extracellular vesicles had a particle size distribution having a peak at approximately 140 nm.

### (4) TEM Observation of Extracellular Vesicles Derived from A375

The left side of (d) of Fig. 13 shows the results of observing, with a TEM, the ultracentrifugation fraction obtained in section (1) above, without labeling. The right side of (d) of Fig. 13 shows the results of observing, with a TEM, the ultracentrifugation fraction obtained in section (1) above, after immunogold labeling. An anti-mouse Alix antibody (available from Santa Cruz Biotechnology) was used as an antibody. As illustrated on the right side of (d) of Fig. 13, it was found that exosomes existed in the ultracentrifugation fraction, at the positions indicated by arrows.

### (5) HPLC Analysis of Surface Sugar Chains of Extracellular Vesicles Derived from A375

An analysis using HPLC was carried out on high-mannose N-type sugar chains appearing on the surface of extracellular vesicles in the ultracentrifugation fraction obtained in section (1) above. First, 100 µg of the ultracentrifugation fraction obtained in section (1) above was reacted with endoglycosidase H (Endo H, available from Roche) 3.8 (v/v)% at 37°C, so that sugar chains were cut from the extracellular vesicles. The sugar chain fraction was collected by ethanol precipitation and then freeze dried.

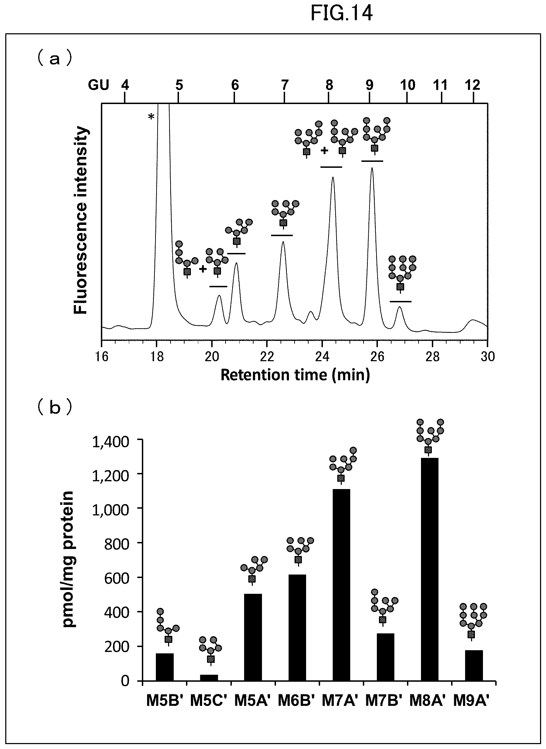

Next, with use of a Blot Glyco Kit (available from Sumitomo Bakelite Co., Ltd.), HPLC analysis was carried out. The analysis used a two-dimensional mapping method in which the sugar chain fraction was labeled with 2-aminopyridine (PA), and a reverse-phase column and anion exchange column were used for the labeled sugar chains.
(a) of Fig. 14 indicates the results of fractionation of the PA-labeled sugar chain via HPLC. Peaks were fractionated. The sugar chain structures indicated in (a) of Fig. 14 were inferred by dual gradient reverse phase HPLC analysis. In (a) of Fig. 14, "GU" denotes glucose units, and the asterisk denotes a peak derived from a reagent used in labeling. In (a) of Fig. 14, peaks corresponding to 8 and 9 are high-mannose sugar chains. From (a) of Fig. 14, it was confirmed that high-mannose sugar chains were the main component of surface sugar chains of extracellular vesicles derived from A375.
(b) of Fig. 14 indicates the results of using dual gradient reverse phase HPLC to evaluate the amount of sugar chains labeled with PA, with use of PA-labled glucose hexamer as an external standard. In (b) of Fig. 14, the horizontal axis represents sugar chain structure, and the vertical axis represents sugar chain amount. From (b) of Fig. 14, it can be seen that on the surface of extracellular vesicles in the ultracentrifugation fraction obtained in section (1) above, (i) the high-mannose sugar chain corresponding to 9 in (a) of Fig. 14 appeared the most, and (ii) the high-mannose sugar chain corresponding to 8 in (a) of Fig. 14 appeared second most.

### (6) Western Blot Analysis of Ultracentrifugation Fraction Treated with Endoglycosidase H

SDS-PAGE was carried out for (i) the ultracentrifugation fraction treated with endoglycosidase H in section (5) above and (ii) a control ultracentrifugation fraction which was treated with PBS instead of endoglycosidase H. Electrophoresis conditions were the same as in Example 1. An SDS polyacrylamide gel that had been subjected to electrophoresis was transferred to a nitrocellulose membrane, and blocking was carried out. Thereafter, the gel was reacted with 5 µg/ml of OAA solution (solvent: 1% BSA/phosphate buffer (pH 7.4) and 0.02% Tween20 (hereinafter referred to as "PBS-T")) at room temperature for 2 hours. In this way, the OAA was reacted with surface sugar chains of the extracellular vesicles.

Next, the nitrocellulose membrane was reacted with mouse anti-6xHis antibody (available from Wako Pure Chemical) at 4°C for 16 hours. After the reaction finished, the nitrocellulose membrane was washed thrice with PBS-T and then reacted (room temperature, 1 hour) with HRP (peroxidase)-labeled sheep anti-mouse antibody (available from GE). The nitrocellulose membrane was then washed thrice with PBS-T and a SuperSignal West Pico Chemiluminescent Substrate (available from Thermo Fisher) was used to achieve chemiluminescence. An Amersham Hyperfilm (available from GE) was used for detection. Also prepared was an ultracentrifugation fraction treated with PNGaseF, instead of the ultracentrifugation fraction treated with the endoglycosidase H in section (5) above. In other words, 0.2 M of 2-mercaptoethanol was added to the ultracentrifugation fraction obtained in section (1) above, and then the ultracentrifugation fraction was subjected to heat denaturation for 3 minutes at 100°C. After cooling, 2.5 (v/v)% PNGaseF was added, and a reaction was carried out for 16 hours at 37°C. In this way, the ultracentrifugation fraction treated with PNGaseF was prepared.

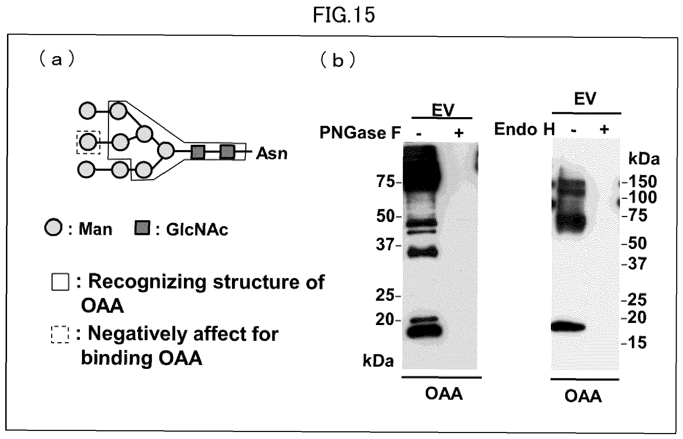

(a) of Fig. 15 illustrates the structure of a high-mannose N-type sugar chain. The high-mannose N-type sugar chain binds to OAA at the portion denoted as "Recognizing structure of OAA" in (a) of Fig. 15. (b) of Fig. 15 indicates results of Western blots. The left side of (b) of Fig. 15 indicates the results for the extracellular vesicles treated with PNGaseF (denoted by a "+" in the figure) and the results for the extracellular vesicles not treated with PNGaseF (denoted by a "-" in the figure). The right side of (b) of Fig. 15 indicates the results for the extracellular vesicles treated with Endo H (denoted by a "+" in the figure) and the results for the extracellular vesicles not treated with Endo H (denoted by a "-" in the figure). In the "-" lanes in (b) of Fig. 15, because enzyme treatment was not carried out, OAA which had bound to high-mannose sugar chains was detected. In contrast, in the "+" lanes in (b) of Fig. 15, high-mannose sugar chains were cut by enzyme treatment, and the high-mannose sugar chains were unable to react with OAA, so no band was detected.

### (7) Confirmation of OAA Binding to Extracellular Vesicles Derived from A375 Dependent on High-Mannose N-Type Sugar Chain

30 µg of the ultracentrifugation fraction treated with endoglycosidase H in section (5) above was incubated (room temperature, 2 hours) along with 1.2 µg of OAA and then subsequently subjected to ultracentrifugation (100,000 g, 70 minutes, 4°C). A resultant precipitate and supernatant (Sup) were subjected to a Western blot with use of (i) mouse anti-6xHis antibody (available from Wako Pure Chemical), (ii) mouse anti-Alix antibody (available from Santa Cruz Biotechnology), (iii) rabbit anti-flotillin-1 antibody (available from Santa Cruz Biotechnology), and (iv) rabbit anti-CD9 antibody (available from SBI). The Western blot was carried out via a procedure similar to that for section (2) above.

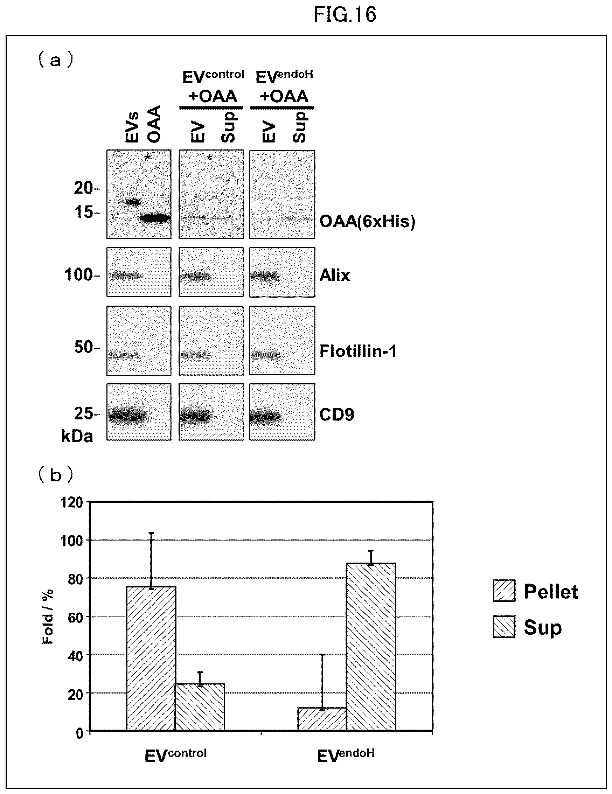

(a) of Fig. 16 indicates results of the Western blot. The leftmost column in (a) of Fig. 16 represents input. The lane denoted as "EVs" and the lane denoted as "OAA" in (a) of Fig. 16 indicate the band positions of each protein.

The center column in (a) of Fig. 16 represents the results of (i) a fraction ("EV" in the figure) prepared from precipitate obtained with use of a ultracentrifugation fraction obtained without endoglycosidase H treatment (instead of the above "ultracentrifugation fraction treated with endoglycosidase H") and (ii) a Sup fraction obtained with use of the ultracentrifugation fraction obtained without endoglycosidase H treatment. In a comparison between the results of the lane denoted as "EV" and the lane denoted as "Sup" in the figure, it can be seen that in the EV fraction, OAA exists in a larger amount, and OAA is bound to extracellular vesicles.

The rightmost column in (a) of Fig. 16 represents the results of (i) a fraction ("EV" in the figure) prepared from precipitate obtained with use of the above "ultracentrifugation fraction treated with endoglycosidase H"(ii) a Sup fraction obtained with use of the above "ultracentrifugation fraction treated with endoglycosidase H". In a comparison between the results of the lane denoted as "EV" and the lane denoted as "Sup" in the figure, it can be seen that the extracellular vesicles treated with endoglycosidase H do not bind with OAA.

(b) of Fig. 16 indicates the results of quantifying the results shown in (a) of Fig. 16. The results shown in (b) of Fig. 16 were obtained with use of the image processing software ImageJ, in accordance with the manual for ImageJ. In (b) of Fig. 16, the graph denoted as "EV control" corresponds to the results shown for "OAA(6xHis)" in the center column of (a) of Fig. 16, and the graph denoted as "EV endoH" corresponds to the results shown for "OAA(6xHis)" in the rightmost column of (a) of Fig. 16. "Pellet" in (b) of Fig. 16 indicates the "EV" of (a) of Fig. 16, and "Sup" in (b) of Fig. 16 indicates the "Sup" of (a) of Fig. 16. The vertical axis in (b) of Fig. 16 represents the collection rate of OAA in each fraction. From the results indicated in (b) of Fig. 16, it can be seen that OAA binds with extracellular vesicles.

### (8) Purification of Extracellular Vesicles Derived from A375 with Use of OAA Solid-Phased Magnetic Beads

Purification of extracellular vesicles derived from A375 was carried out with use of OAA solid-phased magnetic beads instead of the AIST-OAA spin column used in, for example, Example 1. The OAA solid-phased magnetic beads are obtained by coating OAA onto protein G solid-phased magnetic beads.

The left side of (a) of Fig. 17 is a diagram schematically illustrating a method of preparing the OAA solid-phased magnetic beads. First, 40 µg of His-OAA (His-tag OAA) obtained by binding 6x histidine (His) to OAA was mixed with 10 µg of anti-6xHis antibody (available from Wako Pure Chemical) for 2 hours at room temperature. In this way, antibody-OAA complexes (denoted as "His-OAA" in (a) of Fig. 17) were prepared.

Next, the antibody-OAA complexes were mixed with 50 p1 of Protein G solid-phased magnetic beads (available from Thermo Fisher, denoted as "protein G beads" in (a) of Fig. 17) which had been washed beforehand with PBS. A reaction was then allowed to take place for 10 minutes at room temperature. In this way, the complex was coated onto the Protein G solid-phased magnetic beads.

After the reaction finished, the magnetic beads were washed thrice with PBS in order to remove unreacted material. In this way, the OAA solid-phased magnetic beads (denoted as "OAA beads" in (a) of Fig. 17) were obtained.

Note that, as a control, GFP solid-phased magnetic beads (denoted as "GFP beads" in (a) of Fig. 17) were prepared by a method similar to that for OAA, except that GFP was used instead of OAA.

The right side of (a) of Fig. 17 indicates the results of a Western blot of the antibody-OAA complex and the antibody-GFP complex. The Western blot was carried out via a procedure similar to that for section (2) above. When the antibody-OAA complex was submitted to the Western blot (results denoted as "OAA beads" in (a) of Fig. 17), (i) light chains of IgG (band denoted by asterisk) and (ii) His-tag OAA (band denoted as "OAA (His)") were detected. When the antibody-GFP complex was submitted to the Western blot (results denoted as "GFP beads" in (a) of Fig. 17), (i) light chains of IgG (band denoted by asterisk) and (ii) His-tag GFP (band denoted as "GFP (His)") which was obtained by binding 6xhistidine to GFP were detected.

(b) of Fig. 17 indicates results of a pulldown assay of extracellular vesicles derived from A375, carried out with use of OAA solid-phased magnetic beads and GFP solid-phased magnetic beads. In (b) of Fig. 17, the asterisk denotes light chains of IgG.

First, the OAA solid-phased magnetic beads and the GFP solid-phased magnetic beads were each respectively mixed with the ultracentrifugation fraction obtained in section (1) above and then reacted for 16 hours at 4°C. The OAA solid-phased magnetic beads and the GFP solid-phased magnetic beads were each used in an amount of 50 µ1, and the ultracentrifugation fraction was used in an amount of 30 µg.

After the reaction finished, the OAA solid-phased magnetic beads and the GFP solid-phased magnetic beads were collected and then washed thrice with PBS so as to remove non-specific adsorbates. Next, the OAA solid-phased magnetic beads and the GFP solid-phased magnetic beads were each respectively dissolved in a 2x sample buffer (125mM Tris-HCl buffer (pH6.8), 4% SDS, 20% glycerin, 0.02% bromophenol blue). This solution was then reduced while being heated for 5 minutes at 100°C. Thereafter, the OAA solid-phased magnetic beads and the GFP solid-phased magnetic beads were collected. A supernatant of each was then subjected to electrophoresis via SDS-PAGE, under conditions similar to those in Example 1.

A Western blot was carried out using the same conditions as for Example 1, except that the primary antibodies used were (i) mouse anti-Alix antibody (available from Santa Cruz Biotechnology), (ii) rabbit anti-Flotillin-1 antibody (available from Santa Cruz Biotechnology), and (iii) mouse anti-CD81 antibody (available from Santa Cruz Biotechnology).

As indicated in (b) of Fig. 17, in a case where the OAA solid-phased magnetic beads were used, bands were clearly detected for Alix, Flotillin-1 (denoted as "Flot" in (b) of Fig. 17), and CD81. As such, it was found that the OAA solid-phased magnetic beads make it possible to capture extracellular vesicles (exosomes) in the ultracentrifugation fraction.

(c) of Fig. 17 indicates results of blotting, with OAA, components captured by the OAA solid-phased magnetic beads and the GFP solid-phased magnetic beads, after immunoprecipitation (denoted as "IP" in (c) of Fig. 17). The left lane in (c) of Fig. 17 indicates results for the component captured by the GFP solid-phased magnetic beads. The right lane in (c) of Fig. 17 indicates results for the component captured by the OAA solid-phased magnetic beads. The left lane indicates results for the component captured by the GFP solid-phased magnetic beads. The double asterisks in (c) of Fig. 17 indicate His-tag OAA in the right lane and His-tag GFP in the left lane.

From (c) of Fig. 17, it can be seen that glycoproteins recognized by OAA are captured by the OAA solid-phased magnetic beads.

### Industrial Applicability

The present invention is applicable to cancer diagnosis.

### Reference Signs List

1 Cancer diagnosis device
11 Liquid delivery system
12 Plate
13, 15a to 15c, 19a to 19c, 21a to 21c Flow path (first to fourth flow paths)
14 Introduction section
16 to 18 Extracellular vesicle capturing section
16' to 18' Extracellular vesicle capturing section accommodating section
20a to 20c Discharge section
22 MicroRNA accommodating section
23 Waste liquid accommodating section
24 Accommodating section for microRNA extracted from microparticle
25 Accommodation section for microRNA extracted from exosome

## Claims

1. A cancer diagnosis device, comprising:
an extracellular vesicle capturing section including one or more immobilization supports on which one or more kinds of lectins are immobilized respectively, the one or more kinds of lectins being each capable of binding specifically to a surface sugar chain included in an extracellular vesicle derived from a cancer cell, the one or more immobilization supports corresponding respectively to one or more kinds of the surface sugar chain, the extracellular vesicle capturing section being configured to capture the extracellular vesicle through specific binding to a corresponding one of the one or more kinds of lectins; and
a detecting section configured to detect a microRNA included in the extracellular vesicle.

2. The cancer diagnosis device according to claim 1, wherein
the extracellular vesicle is an exosome and/or a microparticle.

3. The cancer diagnosis device according to claim 1 or 2, wherein
the one or more immobilization supports are each a monolithic gel or a lectin-solid-phased magnetic bead.

4. The cancer diagnosis device according to claim 3, wherein
the monolithic gel is a silica monolith.

5. The cancer diagnosis device according to any one of claims 1 to 4, further comprising:
an introduction section configured to introduce a sample into the extracellular vesicle capturing section;
one or more discharge sections each configured to discharge liquid eluted from the extracellular vesicle capturing section and including the microRNA, the extracellular vesicle capturing section being positioned between the introduction section and the one or more discharge sections;
a flow path between the introduction section and the extracellular vesicle capturing section; and
a flow path between the extracellular vesicle capturing section and each of the one or more discharge sections,
wherein the one or more discharge sections correspond in number to the one or more immobilization supports.

6. The cancer diagnosis device according to any one of claims 1 to 5, wherein:
the one or more kinds of lectins are
one or more kinds of high-mannose sugar chain binding lectins in a case where the surface sugar chain is a high-mannose sugar chain,
one or more kinds of sialyl Lewis sugar chain binding lectins in a case where the surface sugar chain is a sialyl Lewis sugar chain, or
one or more kinds of core α1-6 fucose binding lectins in a case where the surface sugar chain is a core α1-6 fucose.

7. The cancer diagnosis device according to claim 6, wherein
the one or more kinds of high-mannose sugar chain binding lectins are one or more kinds of lectins selected from the group consisting of Solnin A, Solnin B, Solnin C, ESA-1, ESA-2, EAA-1, EAA-2, EAA-3, EDA-1, EDA-2, EDA-3, ECA-1 (KAA-1), ECA-2 (KAA-2), KAA-3, KSA-1, KSA-2, MPA-1, MPA-2, Granin-BP, ASL-1, ASL-2, OAA, BCA, BPL17, BML17, BCL17, and MPL-1.

8. The cancer diagnosis device according to claim 6, wherein
the one or more sialyl Lewis sugar chain binding lectins are each a selectin.

9. The cancer diagnosis device according to claim 6, wherein
the one or more kinds of core α1-6 fucose binding lectins are one or more kinds of lectins selected from the group consisting of Hypnin A-1, Hypnin A-2, and Hypnin A-3.

10. The cancer diagnosis device according to any one of claims 1 to 9, wherein:
the extracellular vesicle is included in one or more kinds of samples selected from the group consisting of blood, blood plasma, blood serum, saliva, tear, swab, phlegm, urine, spinal fluid, amniotic fluid, synovial fluid, ascitic fluid, and pleural fluid.
